(19) Europäisches Patentamt
European Patent Office
Office européen des brevets

(11) EP 3 719 003 A1

(12) **EUROPEAN PATENT APPLICATION**
published in accordance with Art. 153(4) EPC

(43) Date of publication:
**07.10.2020 Bulletin 2020/41**

(21) Application number: **18883497.2**

(22) Date of filing: **28.11.2018**

(51) Int Cl.:
***C07C 291/06*** *(2006.01)* ***C08F 8/30*** *(2006.01)*

(86) International application number:
**PCT/JP2018/043868**

(87) International publication number:
**WO 2019/107450 (06.06.2019 Gazette 2019/23)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.11.2017 JP 2017227833**
**28.11.2017 JP 2017227834**

(71) Applicant: **Mitsubishi Chemical Corporation**
**Tokyo 100-8251 (JP)**

(72) Inventors:
- **ITAGAKI Koji**
  **Tokyo 100-8251 (JP)**
- **OHTAKI Hisashi**
  **Tokyo 100-8251 (JP)**
- **TAKATA Toshikazu**
  **Tokyo 152-8550 (JP)**
- **SOGAWA Hiromitsu**
  **Tokyo 152-8550 (JP)**

(74) Representative: **Hoffmann Eitle**
**Patent- und Rechtsanwälte PartmbB**
**Arabellastraße 30**
**81925 München (DE)**

(54) **NITRILE OXIDE COMPOUND, COMPOSITION, MODIFIED POLYOLEFIN AND METHOD FOR PRODUCING SAME, AND METHOD FOR PRODUCING BLOCK COPOLYMER**

(57) A nitrile oxide compound which is a compound represented by General Formula [I], in which a melting point is 25°C to 300°C, and an equivalent of nitrile oxide is 1.0 to 4.5 mmol/g.

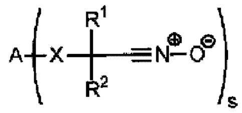

··· [I]

In the general formula, s: an integer of 1 to 4; $R^1$ and $R^2$: a hydrocarbon group having 4 to 10 carbon atoms or a halogenated hydrocarbon group having 4 to 10 carbon atoms; X: a divalent hydrocarbon group, -O-, -S-, or -N($R^3$)-; $R^3$: a hydrogen atom or a hydrocarbon group having 1 to 6 carbon atoms; and A: an s-valent organic group.

EP 3 719 003 A1

## Description

[Technical Field]

The present invention relates to a nitrile oxide compound, a composition, a modified polyolefin and a method for producing the same, and a method for producing a block copolymer.

Priority is claimed on Japanese Patent Application No. 2017-227833, filed November 28, 2017, and Japanese Patent Application No. 2017-227834, filed November 28, 2017, the contents of which are incorporated herein by reference.

[Background Art]

When a nitrile oxide group is heated under uncatalyzed conditions, it undergoes a [2 + 3] cycloaddition reaction with an unsaturated bond easily and without formation of by-product. Accordingly, a nitrile oxide compound is useful as a reactant in various usage applications. For example, the compound is used for crosslinking or modifying diene rubbers. However, a nitrile oxide group is liable to cause side reactions such as dimerization and isomerization, and therefore it is extremely unstable (Non-Patent Literature 1).

As a nitrile oxide compound in which dimerization of a nitrile oxide group is inhibited, an aromatic nitrile oxide compound in which a substituent is introduced at the ortho position of a nitrile oxide group is known (Non-Patent Literature 1).

As a nitrile oxide compound in which isomerization of a nitrile oxide group is inhibited, an aliphatic nitrile oxide compound in which a bulky substituent is introduced into a carbon atom at the $\alpha$-position of a nitrile oxide group is known (Non-Patent Literature 2, Non-Patent Literature 3, Patent Literature 1, Patent Literature 2, and Patent Literature 3).

Polyolefins such as polyethylene and polypropylene have excellent moldability, mechanical properties, and the like, and polyolefins having a wide range of properties from hard to soft properties are produced. Accordingly, usage applications of polyolefins are wide ranging from industrial usage applications to living materials as films, sheets, fibers, non-woven fabrics, molded articles (containers and the like), modifiers, and the like.

It is known that polyolefins have poor reactivity in addition to poor stainability and adhesiveness because polyolefins consist of saturated hydrocarbon skeletons. It is also known that compatibility with other polymers is poor. As a method of solving these problems, a method of modifying a polyolefin with a peroxide is known. However, when this method is used, a crosslinking reaction proceeds in polyethylene, and a decrease in molecular weight occurs in combination therewith as a side reaction in polypropylene.

In recent years, a polyolefin having a carbon-carbon double bond at one end of two ends of a main chain has been reported, and attempts have been made to introduce a polar functional group into the carbon-carbon double bond at the end (Non-Patent Literature 4). As long as a polar functional group can be introduced into the carbon-carbon double bond at the end by a chemical reaction, adhesiveness, stainability, and the like of the polyolefin will be improved. In addition, by reacting the polar functional group introduced at the end with other polymers, it is possible to produce a block copolymer, and adhesion to and compatibility with other polymers are possible.

However, little is known about a reaction itself that may be caused by the carbon-carbon double bond at the end of the polyolefin, and there are only few reactants that can react quickly and selectively with the carbon-carbon double bond at the end of the polyolefin. Accordingly, application examples of the reaction between the polyolefin having the carbon-carbon double bond at the end and a reactant are limited. In addition, the reaction between the polyolefin having the carbon-carbon double bond at the end and a reactant is preferably a reaction at high temperatures from the viewpoint of productivity. However, there are very limited number of reactants that proceed quickly and selectively with the carbon-carbon double bond at the end of the polyolefin at high temperatures. Accordingly, there is a need for a reactant that reacts quickly with the carbon-carbon double bond at the end of the polyolefin, particularly a reactant that reacts therewith at high temperatures.

As a crosslinking agent and a modifier for diene rubbers, a compound having a 1,3-dipolar functional group such as a compound having a nitrile oxide group is known (Patent Literature 4 and Patent Literature 5). Since when a compound having a nitrile oxide group is heated under uncatalyzed conditions, it undergoes a [2 + 3] cycloaddition reaction with an unsaturated bond in another compound easily and without formation of by-product, it is useful as a reactant in various usage applications.

[Citation List]

[Patent Literature]

[Patent Literature 1]
Japanese Unexamined Patent Application, First Publication No. 2016-65033
[Patent Literature 2]
PCT International Publication No. WO2016/143869
[Patent Literature 3]
PCT International Publication No. WO2016/143870
[Patent Literature 4]
Japanese Unexamined Patent Application, First Publication No. Hei 11-180943
[Patent Literature 5]
Japanese Unexamined Patent Application, First Publication No. 2011-52072

[Non-Patent Literature]

[Non-Patent Literature 1]
Koyama, et al., "Nitrile Oxide Agents for Click Reaction: Catalyst-Free Cycloaddition Reaction Involving C-C Bond Formation," Journal of Synthetic Organic Chemistry, Japan, Vol. 47, No. 9, 2016, p. 866-876
[Non-Patent Literature 2]
Ching-Fa Yao, et al., "REACTIONS OF β-NITROTYRENES WITH GRIGNARD REAGENTS," Tetrahedron Letters, Vol. 37, No. 35, 1996, p. 6339-6342
[Non-Patent Literature 3]
Toyokazu Tsutsuba, et al., "Kinetically Stabilized Aliphatic Nitrile N-Oxides as Click Agents: Synthesis, Structure, and Reactivity," Chemistry Letters, Vol. 46, 2017, p. 315-318
[Non-Patent Literature 4]
Patrick Brant, et al., "Termination Events in Sterically Hindered Metallocene-Catalyzed Olefin Oligomerizations: Vinyl Chain Ends in Oligooctenes," Vol. 35, 2016, p. 2836-2839

[Summary of Invention]

[Technical Problem]

However, when a nitrile oxide group in an aromatic nitrile oxide compound disclosed in Non-Patent Literature 1 is heated, it is easily irreversibly isomerized to an isocyanate group, and therefore it cannot react with an unsaturated bond.

Non-Patent Literature 2 discloses an aliphatic nitrile oxide compound having one alkyl group and one nitrile oxide group in the skeleton. However, because a functional group which is not a nitrile oxide group is an alkyl group (a hydrocarbon group), even when this compound is added to a polymer consisting of a hydrocarbon skeleton represented by polyolefins, functions such as colorability, stainability, and reactivity cannot be imparted to the polymer.

Non-Patent Literature 3, Patent Literature 1, Patent Literature 2, and Patent Literature 3 disclose aliphatic nitrile oxide compounds produced using oxygen nucleophiles which are inexpensive and easily handled.

However, according to studies by the inventors of the present invention, dimerization of nitrile oxide groups proceeds during storage of these aliphatic nitrile oxide compounds, and this makes stability of the compounds insufficient.

There is a strong demand for a nitrile oxide compound which can be stably stored without dimerization of a nitrile oxide group even at room temperature while still maintaining high reactivity of the nitrile oxide group, and which is easily synthesized without isomerization of a nitrile oxide group to an isocyanate group even at high temperatures.

In addition, until now there has been no report that a compound having a 1,3-dipolar functional group is reacted with a carbon-carbon double bond at one end of two ends of a main chain of a polyolefin at high temperatures.

The present invention provides a nitrile oxide compound which is easily synthesized, has high reactivity, but still has excellent storage stability at room temperature, and in which a nitrile oxide group is hardly isomerized even at a high temperature; and a composition including the nitrile oxide compound.

The present invention further provides a method for modifying a polyolefin with a compound having a 1,3-dipolar functional group under industrially advantageous high-temperature conditions, and a method for producing a block copolymer using the obtained modified polyolefin.

[Solution to Problem]

The inventors of the present invention have studied improvement of nitrile oxide compounds in order to achieve

the above-mentioned objects, and have found that the above-mentioned objects can be achieved by a novel nitrile oxide compound which has a specific structure, is solid at room temperature, and has an equivalent of nitrile oxide within a specific range, that is, a compound which is easily synthesized, has high reactivity, but still has excellent storage stability at room temperature, and in which a nitrile oxide group is hardly isomerized even at a high temperature. Thereby, they have completed the present invention.

The inventors of the present invention have conducted various studies and experimental searches to achieve the above-mentioned objects, and have found that, by using a polyolefin having a specific structure and a compound having a 1,3-dipolar functional group, it is possible to produce a modified polyolefin at a high modification temperature that is industrially advantageous, and to produce a block copolymer by using the modified polyolefin. Thereby, they have completed the present invention.

The present invention has the following first aspect.

<1> A nitrile oxide compound which is represented by General Formula [I], having a melting point of 25°C to 300°C, and having an equivalent of nitrile oxide of 1.0 to 4.5 mmol/g.

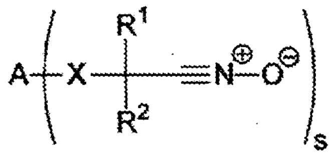

··· [I]

In General Formula [I], s is an integer of 1 to 4; $R^1$ and $R^2$ are each independently a hydrocarbon group having 4 to 10 carbon atoms or a halogenated hydrocarbon group having 4 to 10 carbon atoms; X is -O-, -S-, or -N($R^3$)-; $R^3$ is a hydrogen atom or a hydrocarbon group having 1 to 6 carbon atoms; and A is an s-valent organic group.

<2> The nitrile oxide compound according to <1>, in which, in General Formula [I], $R^1$ and $R^2$ are each independently an aryl group having 6 to 8 carbon atoms.

<3> The nitrile oxide compound according to <1> or <2>, in which, in General Formula [I], s is 2, and A is an alkylene group having 2 to 10 carbon atoms.

<4> The nitrile oxide compound according to <3>, in which, in General Formula [I], A is a 1,2-ethylene group, a 1,3-propylene group, a 2-methyl-1,3-propylene group, a 2,2-dimethyl-1,3-propylene group, a 1,4-butylene group, a 1,5-pentylene group, a 1,6-hexylene group, a 1,7-heptylene group, a 1,8-octylene group, a 3-methyl-1,5-pentylene group, a 1,4-cyclohexylene group, a 1,4-cyclohexadimethylene group, a 1-methyl-1,2-ethylene group, or a 1-methyl-1,3-propylene group.

<5> The nitrile oxide compound according to <1> or <2>, in which, in General Formula [I], s is 2, and A is a group represented by General Formula [II].

$$-(R^4\text{-}O)_m\text{-}R^5\text{-}(O\text{-}R^4)_m\text{-} \qquad [II]$$

In General Formula [II], m is 0 or 1, $R^4$ is an alkylene group having 2 to 4 carbon atoms, and $R^5$ is a group represented by General Formula [III] or a group represented by General Formula [IV].

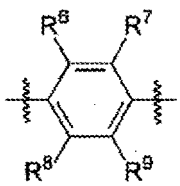

··· [III]

In General Formula [III], $R^6$ to $R^9$ are each independently a hydrogen atom, a hydrocarbon group having 1 to 6 carbon atoms, or a halogen atom, $R^6$ and $R^7$ may be linked to form an aromatic ring or an aliphatic ring, and $R^8$ and $R^9$ may be linked to form an aromatic ring or an aliphatic ring.

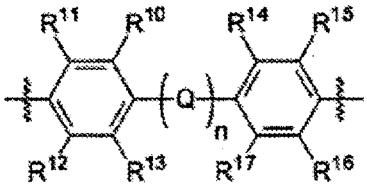

...[IV]

In General Formula [IV], $R^{10}$ to $R^{17}$ are each independently a hydrogen atom, a hydrocarbon group having 1 to 6 carbon atoms, or a halogen atom, $R^{10}$ and $R^{11}$ may be linked to form an aromatic ring or an aliphatic ring, $R^{12}$ and $R^{13}$ may be linked to form an aromatic ring or an aliphatic ring, $R^{14}$ and $R^{15}$ may be linked to form an aromatic ring or an aliphatic ring, and $R^{16}$ and $R^{17}$ may be linked to form an aromatic ring or an aliphatic ring; n is 0 or 1; and Q is $-C(R^{18})(R^{19})-$, -C(=O)-, -S-, or $-S(=O)_2-$, where $R^{18}$ and $R^{19}$ are each independently a hydrogen atom, a hydrocarbon group having 1 to 6 carbon atoms, or a halogen atom, and $R^{18}$ and $R^{19}$ may be linked to form an aromatic ring or an aliphatic ring.

<6> The nitrile oxide compound according to <5>, in which, in General Formula [II], m is 1, and $R^5$ is a group represented by General Formula [IV]; and in General Formula [IV], n is 1, and Q is $-C(R^{18})(R^{19})-$.

<7> The nitrile oxide compound according to <1> or <2>, in which, in General Formula [I], s is 1, and A is a group represented by General Formula [V].

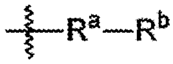

...[V]

In General Formula [V], $R^a$ is an alkylene group having 1 to 5 carbon atoms or an arylene group having 6 to 10 carbon atoms, and $R^b$ is a polar functional group.

<8> The nitrile oxide compound according to <7>, in which, in General Formula [V], $R^b$ is a hydroxy group, a mercapto group, a carboxy group, an amino group, an amino group having a substituent, an amide group, $-OR^{20}$ (where $R^{20}$ is an alkyl group or an aryl group), or a heterocyclic ring.

<9> A composition including: the nitrile oxide compound according to any one of <1> to <8>; and a substance that reacts with a nitrile oxide group of the nitrile oxide compound.

<10> The composition according to <9>, in which the substance that reacts with a nitrile oxide group is a resin or a rubber.

<11> The composition according to <10>, in which the resin or the rubber has at least one bond selected from the group consisting of a carbon-carbon double bond, a carbon-carbon triple bond, a carbon-nitrogen double bond, and a carbon-nitrogen triple bond.

In addition, the present invention has the following second aspect.

<1> A method for producing a modified polyolefin, the method including performing an addition reaction of a polyolefin having a carbon-carbon double bond at one end of two ends of a main chain with a compound having a 1,3-dipolar functional group.

<2> The method for producing a modified polyolefin according to <1>, in which the compound having a 1,3-dipolar functional group is a nitrile oxide compound.

<3> The method for producing a modified polyolefin according to <2>, in which the nitrile oxide compound is a compound represented by General Formula [I].

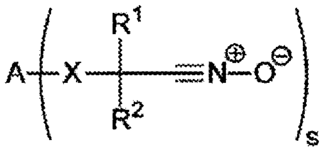

...[I]

In General Formula [I], s is an integer of 1 to 4; $R^1$ and $R^2$ are each independently a hydrocarbon group having 4 to 10 carbon atoms or a halogenated hydrocarbon group having 4 to 10 carbon atoms; X is a divalent hydrocarbon group, -O-, -S-, or $-N(R^3)-$; $R^3$ is a hydrogen atom or a hydrocarbon group having 1 to 6 carbon atoms; and A is an s-valent organic group.

<4> The method for producing a modified polyolefin according to any one of <1> to <3>, in which the number of

carbon-carbon double bonds per one polyolefin molecule in the polyolefin is 0.1 to 4.0 bonds/molecule.
<5> The method for producing a modified polyolefin according to any one of <1> to <4>, in which the carbon-carbon double bond at the end of the main chain of the polyolefin is a vinyl group or a vinylidene group.
<6> The method for producing a modified polyolefin according to any one of <1> to <5>, further including performing an addition reaction of the polyolefin with the compound having a 1,3-dipolar functional group under conditions substantially free of a solvent.
<7> A modified polyolefin obtained by the production method according to any one of <1> to <6>.
<8> A modified polyolefin having a structure represented by General Formula [VIII].

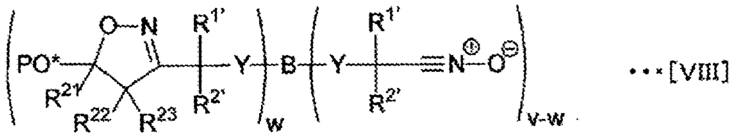

In General Formula [VIII], v is an integer of 1 to 4; w is an integer of 1 to 4; $v \geq w$; PO* is a main chain of the polyolefin; $R^{21}$, $R^{22}$, and $R^{23}$ are each independently a hydrogen atom or an alkyl group having 1 to 8 carbon atoms; $R^{1'}$ and $R^{2'}$ are each independently a hydrocarbon group having 4 to 10 carbon atoms or a halogenated hydrocarbon group having 4 to 10 carbon atoms; Y's are each independently a divalent hydrocarbon group, -O-, -S-, or -N($R^{3'}$)-; $R^{3'}$ is a hydrogen atom or a hydrocarbon group having 1 to 6 carbon atoms; and B is a v-valent organic group.
<9> The modified polyolefin according to <8>, in which, in General Formula [VIII], $R^{22}$ and $R^{23}$ are hydrogen atoms.
<10> A method for producing a block copolymer, the method including reacting the modified polyolefin according to any one of <7> to <9> with a polymer having a functional group that reacts with a functional group of the modified polyolefin.
<11> The method for producing a block copolymer according to <10>, in which the functional group that reacts with a functional group of the modified polyolefin is at least one selected from the group consisting of a carbon-carbon double bond, a carbon-carbon triple bond, a carbon-nitrogen triple bond, an acid anhydride group, and an amino group.
<12> The method for producing a block copolymer according to <10> or <11>, the method including reacting the modified polyolefin with the polymer under conditions substantially free of a solvent.
<13> The method for producing a block copolymer according to any one of <10> to <12>, in which a reaction temperature is 150°C or higher.

Furthermore, the present invention has the following third aspect.

<1> A nitrile oxide compound which is a compound represented by General Formula [I], in which a melting point is 25°C to 300°C, and an equivalent of nitrile oxide is 1.0 to 4.5 mmol/g.

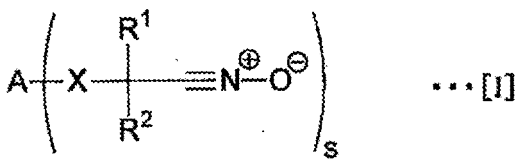

In General Formula [I], s is an integer of 1 to 4; $R^1$ and $R^2$ are each independently a hydrocarbon group having 4 to 10 carbon atoms or a halogenated hydrocarbon group having 4 to 10 carbon atoms; X is a divalent hydrocarbon group, -O-, -S-, or -N($R^3$)-; $R^3$ is a hydrogen atom or a hydrocarbon group having 1 to 6 carbon atoms; and A is an s-valent organic group.
<2> The nitrile oxide compound according to <1>, in which, in General Formula [I], $R^1$ and $R^2$ are each independently an aryl group having 6 to 8 carbon atoms.
<3> The nitrile oxide compound according to <1> or <2>, in which, in General Formula [I], s is 2, and A is an alkylene group having 2 to 10 carbon atoms.
<4> The nitrile oxide compound according to <3>, in which, in General Formula [1], A is a 1,2-ethylene group, a 1,3-propylene group, a 2-methyl-1,3-propylene group, a 2,2-dimethyl-1,3-propylene group, a 1,4-butylene group, a 1,5-pentylene group, a 1,6-hexylene group, a 1,7-heptylene group, a 1,8-octylene group, a 3-methyl-1,5-pentylene group, a 1,4-cyclohexylene group, a 1,4-cyclohexadimethylene group, a 1-methyl-1,2-ethylene group, or a 1-methyl-1,3-propylene group.

<5> The nitrile oxide compound according to <1> or <2>, in which, in General Formula [I], s is 2, and A is a group represented by General Formula [II].

$$-(R^4\text{-}O)_m\text{-}R^5\text{-}(O\text{-}R^4)_m\text{-} \qquad [II]$$

In General Formula [II], m is 0 or 1, $R^4$ is an alkylene group having 2 to 4 carbon atoms, and $R^5$ is a group represented by General Formula [III] or a group represented by General Formula [IV].

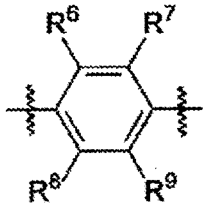

··· [III]

In General Formula [III], $R^6$ to $R^9$ are each independently a hydrogen atom, a hydrocarbon group having 1 to 6 carbon atoms, or a halogen atom, where $R^6$ and $R^7$ may be linked to form an aromatic ring or an aliphatic ring, and $R^8$ and $R^9$ may be linked to form an aromatic ring or an aliphatic ring.

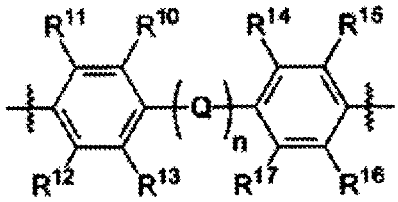

··· [IV]

In General Formula [IV], $R^{10}$ to $R^{17}$ are each independently a hydrogen atom, a hydrocarbon group having 1 to 6 carbon atoms, or a halogen atom, where $R^{10}$ and $R^{11}$ may be linked to form an aromatic ring or an aliphatic ring, $R^{12}$ and $R^{13}$ may be linked to form an aromatic ring or an aliphatic ring, $R^{14}$ and $R^{15}$ may be linked to form an aromatic ring or an aliphatic ring, and $R^{16}$ and $R^{17}$ may be linked to form an aromatic ring or an aliphatic ring; n is 0 or 1; Q is $-C(R^{18})(R^{19})-$, -C(=O)-, -S-, or $-S(=O)_2-$; and $R^{18}$ and $R^{19}$ are each independently a hydrogen atom, a hydrocarbon group having 1 to 6 carbon atoms, or a halogen atom, where $R^{18}$ and $R^{19}$ may be linked to form an aromatic ring or an aliphatic ring.

<6> The nitrile oxide compound according to <5>, in which, in General Formula [II], m is 1, and $R^5$ is a group represented by General Formula [IV]; and in General Formula [IV], n is 1, and Q is $-C(R^{18})(R^{19})-$.

<7> The nitrile oxide compound according to <1> or <2>, in which, in General Formula [I], s is 1, and A is a group represented by General Formula [V].

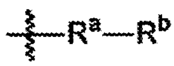

··· [V]

In General Formula [V], $R^a$ is an alkylene group having 1 to 5 carbon atoms or an arylene group having 6 to 10 carbon atoms, and $R^b$ is a polar functional group.

<8> The nitrile oxide compound according to <7>, in which, in General Formula [V], $R^b$ is a hydroxy group, a mercapto group, a carboxy group, an amino group, an amino group having a substituent, an amide group, $-OR^{20}$ (where $R^{20}$ is an alkyl group or an aryl group), or a heterocyclic ring.

<9> A composition including: the nitrile oxide compound according to any one of <1> to <8>; and a substance that reacts with a nitrile oxide group of the nitrile oxide compound.

<10> The composition according to <9>, in which the substance that reacts with a nitrile oxide group is a resin or a rubber.

<11> The composition according to <10>, in which the resin or the rubber has at least one bond selected from the group consisting of a carbon-carbon double bond, a carbon-carbon triple bond, a carbon-nitrogen double bond, and a carbon-nitrogen triple bond.

<12> The composition according to <10> or <11>, in which a content of the nitrile oxide compound is preferably

0.001 % to 10% by mass and more preferably 0.01% to 8% by mass with respect to a total mass of the composition.

<13> The composition according to any one of <10> to <12>, in which a content of the substance that reacts with a nitrile oxide group is preferably 1% to 99.999% by mass and more preferably 20% to 99.9% by mass with respect to the total mass of the composition.

<14> A method for producing a modified polyolefin, the method including performing an addition reaction of a polyolefin having a carbon-carbon double bond at one end of two ends of a main chain with a compound having a 1,3-dipolar functional group.

<15> The method for producing a modified polyolefin according to <14>, in which the compound having a 1,3-dipolar functional group is a nitrile oxide compound.

<16> The method for producing a modified polyolefin according to <15>, in which the nitrile oxide compound is a compound represented by General Formula [I].

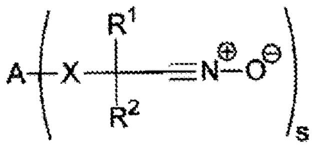

$\cdots$ [I]

In General Formula [I], s is an integer of 1 to 4; $R^1$ and $R^2$ are each independently a hydrocarbon group having 4 to 10 carbon atoms or a halogenated hydrocarbon group having 4 to 10 carbon atoms; X is a divalent hydrocarbon group, -O-, -S-, or $-N(R^3)-$; $R^3$ is a hydrogen atom or a hydrocarbon group having 1 to 6 carbon atoms; and A is an s-valent organic group.

<17> The method for producing a modified polyolefin according to any one of <14> to <16>, in which the number of carbon-carbon double bonds per one polyolefin molecule in the polyolefin is 0.1 to 4.0 bonds/molecule.

<18> The method for producing a modified polyolefin according to any one of <14> to <17>, in which the carbon-carbon double bond at the end of the main chain of the polyolefin is a vinyl group or a vinylidene group.

<19> The method for producing a modified polyolefin according to any one of <14> to <18>, further including performing an addition reaction of the polyolefin with the compound having a 1,3-dipolar functional group under conditions substantially free of a solvent.

<20> The method for producing a modified polyolefin according to <15>, in which the nitrile oxide compound is a compound according to any one of <1> to <8>.

<21> The method for producing a modified polyolefin according to any one of <14> to <20>, in which a blending amount of the compound having a 1,3-dipolar functional group is preferably 0.001 to 10 parts by mass, more preferably 0.01 to 8 parts by mass, and even more preferably 0.05 to 6 parts by mass with respect to 100 parts by mass of the polyolefin.

<22> A modified polyolefin obtained by the production method according to any one of <14> to <21>.

<23> A modified polyolefin having a structure represented by General Formula [VIII].

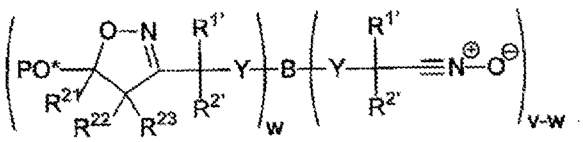

$\cdots$ [VIII]

In General Formula [VIII], v is an integer of 1 to 4; w is an integer of 1 to 4; $v \geq w$; PO* is a main chain of the polyolefin; $R^{21}$, $R^{22}$, and $R^{23}$ are each independently a hydrogen atom or an alkyl group having 1 to 8 carbon atoms; $R^{1'}$ and $R^{2'}$ are each independently a hydrocarbon group having 4 to 10 carbon atoms or a halogenated hydrocarbon group having 4 to 10 carbon atoms; Y's are each independently a divalent hydrocarbon group, -O-, -S-, or $-N(R^{3'})-$; $R^{3'}$ is a hydrogen atom or a hydrocarbon group having 1 to 6 carbon atoms; and B is a v-valent organic group.

<24> The modified polyolefin according to <23>, in which, in General Formula [VIII], $R^{22}$ and $R^{23}$ are hydrogen atoms.

<25> A method for producing a block copolymer, the method including reacting the modified polyolefin according to any one of <22> to <24> with a polymer having a functional group that reacts with a functional group of the modified polyolefin.

<26> The method for producing a block copolymer according to <25>, in which the functional group that reacts with a functional group of the modified polyolefin is at least one selected from the group consisting of a carbon-carbon double bond, a carbon-carbon triple bond, a carbon-nitrogen triple bond, an acid anhydride group, and an amino group.

<27> The method for producing a block copolymer according to <25> or <26>, the method including reacting the

modified polyolefin with the polymer under conditions substantially free of a solvent.

<28> The method for producing a block copolymer according to any one of <25> to <27>, in which a reaction temperature is 150°C or higher.

<29> The method for producing a block copolymer according to <25>, in which a blending amount of the polymer is 10 to 900 parts by mass with respect to 100 parts by mass of the modified polyolefin.

<30> The method for producing a block copolymer according to any one of <25> to <29>, in which the modified polyolefin is reacted with the polymer under a condition in which a proportion of a solvent is 1 part by mass or less with respect to a total of 100 parts by mass of the modified polyolefin and the polymer.

<31> The method for producing a block copolymer according to <30>, in which the solvent is a hydrocarbon.

[Advantageous Effects of Invention]

A nitrile oxide compound of the present invention is a compound which is easily synthesized, has high reactivity, but still has excellent storage stability at room temperature, and in which a nitrile oxide group is hardly isomerized even at a high temperature.

A composition of the present invention has excellent storage stability at room temperature even though the nitrile oxide compound has high reactivity. In addition, since the nitrile oxide group is not isomerized even at a high temperature, the composition of the present invention is excellent in reactivity between the nitrile oxide compound at a high temperature and a substance that may react with the nitrile oxide group of the nitrile oxide compound.

According to a method for producing a modified polyolefin of the present invention, it is possible to modify a polyolefin with a compound having a 1,3-dipolar functional group under industrially advantageous high-temperature conditions.

According to a modified polyolefin of the present invention, it is possible to easily produce a block copolymer having excellent mechanical properties and the like.

According to a method for producing a block copolymer of the present invention, it is possible to easily produce a block copolymer having excellent mechanical properties and the like.

[Brief Description of Drawings]

Fig. 1 shows a $^1$H-NMR spectrum of a modified polyolefin of Example 14B.

Fig. 2 shows a $^1$H-NMR spectrum of a block copolymer of Example 33B.

[Description of Embodiments]

The following definitions of terms apply throughout the present specification and claims.

A "melting point" is a temperature at a time point when a temperature of a sample is raised at a condition of 1 °C/min using a melting point measuring device, and all solid parts are melted.

An "equivalent of nitrile oxide" is an equivalent of a nitrile oxide functional group per unit mass contained in a nitrile oxide compound, and it is determined by the following equation.

$$\text{Equivalent of nitrile oxide [mmol/g]} = 1000 \times (\text{number of nitrile oxide groups in molecule/molecular weight of nitrile oxide compound})$$

The term "to" indicating a numerical value range means that numerical values described before and after the numerical value range are included as the lower limit value and the upper limit value.

The following definitions of terms apply throughout the present specification and claims.

"Polyethylene" is an ethylene homopolymer or ethylene-α-olefin copolymer having a proportion of ethylene units of more than 50 mol%. The polyethylene may have 1 mol% or less of diene compound units.

"Polypropylene" is a propylene homopolymer or propylene-α-olefin copolymer having a proportion of propylene units of more than 50 mol%. The polypropylene may have 1 mol% or less of diene compound units.

A "1,3-dipolar functional group" refers to a functional group that may undergo a 1,3-dipolar cycloaddition reaction with an unsaturated bond (a carbon-carbon double bond, a carbon-carbon triple bond, a carbon-nitrogen triple bond, and the like).

A "main chain" refers to a linear molecular chain in which all molecular chains other than the main chain are

shown as a pendant-like shape.

The phrase "substantially free of a solvent" means that there is no solvent present other than a solvent unavoidably mixed in during production. The phrase "substantially free of a solvent" means that a proportion of a solvent is 1 part by mass or less with respect to a total of 100 parts by mass of a modified polyolefin and the polymer. Specific examples of "solvents" referred to include hydrocarbon solvents such as hexane, heptane, octane, toluene, and xylene, and polar solvents such as acetone, methanol, and ethanol.

"A mass average molecular weight and a number average molecular weight of a polyolefin" are molecular weights obtained by gel permeation chromatography (GPC).

"The number of carbon-carbon double bonds per one polyolefin molecule" is obtained from the following equation.

Number of carbon-carbon double bonds per one polyolefin molecule

[bonds/molecule] = number of carbon-carbon double bonds per 1 g of polyolefin

[mol/g]/number average molecular weight of polyolefin [g/mol]

The number of carbon-carbon double bonds per 1 g of polyolefin is a value obtained from $^1$H-NMR and $^{13}$C-NMR, and a number average molecular weight of polyolefin is a molecular weight obtained by GPC.

The term "to" indicating a numerical value range means that numerical values described before and after the numerical value range are included as the lower limit value and the upper limit value.

<Nitrile oxide compound>

A nitrile oxide compound of the present invention has a nitrile oxide group that is hardly dimerized and isomerized, and therefore the compound has a specific structure represented by General Formula [I].

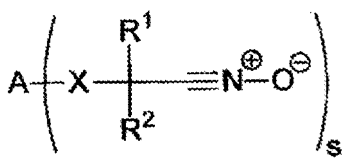

・・・[I]

s is an integer of 1 to 4. s is preferably an integer of 1 to 3 and more preferably 1 or 2 from the viewpoint that the nitrile oxide group is then hardly dimerized in a molecule.

$R^1$ and $R^2$ are each independently a hydrocarbon group having 4 to 10 carbon atoms or a halogenated hydrocarbon group having 4 to 10 carbon atoms. Examples of hydrocarbon groups having 4 to 10 carbon atoms or halogenated hydrocarbon groups having 4 to 10 carbon atoms include a tert-butyl group, an isobutyl group, a phenyl group, a 2-methylphenyl group, a 3-methylphenyl group, a 4-methylphenyl group, a 4-chlorophenyl group, a 2,4-dimethylphenyl group, a 3,4-dimethylphenyl group, and the like.

For $R^1$ and $R^2$, an aryl group having 6 to 8 carbon atoms is preferable from the viewpoint that then, a melting point of the nitrile oxide compound is easily increased, and the nitrile oxide group is hardly dimerized. Examples of aryl groups having 6 to 8 carbon atoms include a phenyl group, a 2-methylphenyl group, a 3-methylphenyl group, a 4-methylphenyl group, a 2,4-dimethylphenyl group, a 4-chlorophenyl group, and the like. Among the examples, a phenyl group, a 2-methylphenyl group, a 3-methylphenyl group, a 4-methylphenyl group, and a 2,4-dimethylphenyl group are preferable, and a phenyl group is more preferable.

$R^1$ and $R^2$ may be the same as or different from each other. $R^1$ and $R^2$ are preferably the same as each other from the viewpoint that then, molecular symmetry is increased, the nitrile oxide compound is easily solidified, and storage stability at room temperature is excellent.

X is a divalent hydrocarbon group, -O-, -S-, or -N($R^3$)-.

X is preferably -O- or -S-, and more preferably -O- from the viewpoint that synthesis of the nitrile oxide compound is then further easier. In addition, X is preferably a divalent hydrocarbon group from the viewpoint of heat resistance.

Examples of divalent hydrocarbon groups for X include an alkylene group having 1 to 3 carbon atoms, an arylene group having 6 to 8 carbon atoms, and a combination thereof. Among the examples, a methylene group is preferable.

$R^3$ is a hydrogen atom or a hydrocarbon group having 1 to 6 carbon atoms. Examples of hydrocarbon groups having 1 to 6 carbon atoms include a methyl group, an ethyl group, a propyl group, a butyl group, a hexyl group, and the like. For $R^3$, a hydrogen atom or a methyl group is preferable from the viewpoint that synthesis of the nitrile oxide compound is then further easier.

A is an s-valent organic group. An organic group essentially has a carbon atom, and has a hydrogen atom, an oxygen atom, a chlorine atom, a nitrogen atom, a sulfur atom, or the like as necessary. Examples of organic groups include a hydrocarbon group (an alkylene group, an arylene group, and the like); a combination of a hydrocarbon group and various bonds (-O-, -C(=O)-, -S-, $-S(=O)_2-$, and the like); a combination of a hydrocarbon group and a polar functional group (a hydroxy group, a mercapto group, a carboxy group, an amino group, an amide group, an alkoxy group, and the like); a combination of a hydrocarbon group, various bonds, and a polar functional group; and the like.

Among the examples, in a case where s = 1, A preferably contains at least a heteroatom such as an oxygen atom, a chlorine atom, a nitrogen atom, and a sulfur atom, or has a polar functional group, from the viewpoint that then, functions such as adhesiveness, stainability, and reactivity are imparted in a case of adding the nitrile oxide compound of the present invention to a polymer consisting of a hydrocarbon skeleton represented by polyolefins. Specific preferable combinations are described below.

In a case of s = 1, $R^1$ and $R^2$ are each independently preferably an aryl group having 6 to 8 carbon atoms; X is preferably a divalent hydrocarbon group, -O-, -S-, or - $N(R^3)$-; and A is preferably a divalent organic group containing at least an oxygen atom, a nitrogen atom, and a sulfur atom.

In a case of s = 2 to 4, $R^1$ and $R^2$ are each independently preferably an aryl group having 6 to 8 carbon atoms; X is preferably a divalent hydrocarbon group, -O-, -S-, or $-N(R^3)-$; and A is preferably a divalent organic group.

The above case in which s is an integer of 2 or more is preferable because of the following reason. In this case, there are two or more nitrile oxide groups, and even in a case where A is a hydrocarbon group, and one nitrile oxide group reacts and loses its reactivity, the remaining one or more nitrile oxide groups have reactivity, and therefore functions such as adhesiveness, stainability, and reactivity can be imparted.

In a case where s = 2 to 4 and X is -O-, $R^1$ and $R^2$ are each independently preferably an aryl group having 6 to 8 carbon atoms, and A is preferably a divalent organic group.

Furthermore, in a case where s = 2 to 4 and X is a divalent hydrocarbon group, $R^1$ and $R^2$ are each independently preferably an aryl group having 6 to 8 carbon atoms, and A is preferably a divalent organic group.

In addition, the nitrile oxide compound of the present invention is preferably the following nitrile oxide compounds (i) to (iii) from the viewpoint that then, a melting point of a nitrile oxide compound is easily increased, and storage stability at room temperature is excellent.

(i) A nitrile oxide compound in which, in General Formula [I], s is 2, and A is an alkylene group having 2 to 10 carbon atoms.

(ii) A nitrile oxide compound in which, in General Formula [I], s is 2, and A is a group represented by General Formula [II] to be shown later.

(iii) A nitrile oxide compound in which, in General Formula [I], s is 1, and A is a group represented by General Formula [V] to be shown later.

It is possible to increase a melting point of a nitrile oxide compound by introducing an alkylene group having high symmetry and a short carbon chain as described in (i).

It is possible to increase a melting point of a nitrile oxide compound by introducing the group represented by General Formula [II] which has a rigid arylene group having high symmetry as described in (ii).

It is possible to increase a melting point of a nitrile oxide compound by introducing the group represented by General Formula [V] which has an alkylene group having a short chain length or a rigid arylene group as described in (iii).

A in (i) is an alkylene group having 2 to 10 carbon atoms. For A in (i), an alkylene group having 3 to 8 carbon atoms is preferable, and an alkylene group having 4 to 6 carbon atoms is more preferable from the viewpoint that then, a melting point at room temperature or higher is exhibited, a nitrile oxide compound is thus solidified, and thereby a melting point close to that of polyolefins is exhibited.

Examples of A in (i) include a 1,2-ethylene group, a 1,3-propylene group, a 2-methyl-1,3-propylene group, a 2,2-dimethyl-1,3-propylene group, a 1,4-butylene group, a 1,5-pentylene group, a 1,6-hexylene group, a 1,7-heptylene group, a 1,8-octylene group, a 3-methyl-1,5-pentylene group, a 1,4-cyclohexylene group, a 1,4-cyclohexadimethylene group, a 1-methyl-1,2-ethylene group, a 1-methyl-1,3-propylene group, and the like. For A in (i), a 1,3-propylene group, a 1,4-butylene group, a 1,6-hexylene group, a 1,4-cyclohexadimethylene group, a 1,4-cyclohexylene group, and a 3-methyl-1,5-pentylene group are preferable; and a 1,4-butylene group, a 1,6-hexylene group, and a 3-methyl-1,5-pentylene group are more preferable.

A in (ii) is the group represented by General Formula [II].

$-(R^4-O)_m-R^5-(O-R^4)_m-$ [II]

m is 0 or 1. m is preferably 1 from the viewpoint of easy production of a nitrile oxide compound, and m is preferably 0 from the viewpoint of a melting point of a nitrile oxide compound.

$R^4$ is an alkylene group having 2 to 4 carbon atoms. Examples of $R^4$ include a 1,2-ethylene group, a 1,3-propylene group, and the like. $R^4$ is preferably a 1,2-ethylene group from the viewpoint that a melting point of a nitrile oxide compound is increased as the number of carbon atoms becomes smaller.

$R^5$ is a group represented by General Formula [III] or a group represented by General Formula [IV]. $R^5$ is preferably the group represented by General Formula [IV] from the viewpoint that then, an appropriate distance is exhibited between crosslinking points when a rubber is used as a crosslinking agent. $R^5$ is preferably General Formula [III] from the viewpoint that then, a higher melting point of a nitrile oxide compound is exhibited. In addition, $R^5$ is preferably the group represented by General Formula [IV] from the viewpoint that then, a distance between an end portion of a modified polyolefin and a functional group being introduced is increased, and thereby reactivity of the modified polyolefin is increased.

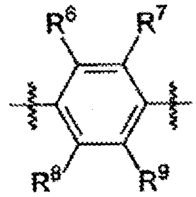

・・・[III]

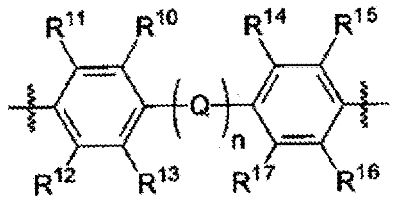

・・・[IV]

$R^6$ to $R^9$ are each independently a hydrogen atom, a hydrocarbon group having 1 to 6 carbon atoms, or a halogen atom, where $R^6$ and $R^7$ may be linked to form an aromatic ring or an aliphatic ring, and $R^8$ and $R^9$ may be linked to form an aromatic ring or an aliphatic ring. Examples of hydrocarbon groups having 1 to 6 carbon atoms include a methyl group, an ethyl group, a propyl group, a butyl group, a hexyl group, a cyclohexyl group, a phenyl group, and the like. Examples of halogen atoms include a fluorine atom, a chlorine atom, a bromine atom, an iodine atom, and the like. For $R^6$ to $R^9$, a hydrogen atom, a methyl group, an ethyl group, an isopropyl group, a tert-butyl group, a phenyl group, and a chlorine atom are preferable; a hydrogen atom, a methyl group, an isopropyl group, and a tert-butyl group are more preferable; and a hydrogen atom and a methyl group are even more preferable.

$R^{10}$ to $R^{17}$ are each independently a hydrogen atom, a hydrocarbon group having 1 to 6 carbon atoms, or a halogen atom, where $R^{10}$ and $R^{11}$ may be linked to form an aromatic ring or an aliphatic ring, $R^{12}$ and $R^{13}$ may be linked to form an aromatic ring or an aliphatic ring, $R^{14}$ and $R^{15}$ may be linked to form an aromatic ring or an aliphatic ring, and $R^{16}$ and $R^{17}$ may be linked to form an aromatic ring or an aliphatic ring. Examples of hydrocarbon groups having 1 to 6 carbon atoms include a methyl group, an ethyl group, a propyl group, a butyl group, a hexyl group, a cyclohexyl group, a phenyl group, and the like. Examples of halogen atoms include a fluorine atom, a chlorine atom, a bromine atom, an iodine atom, and the like. For $R^{10}$ to $R^{17}$, a hydrogen atom, a methyl group, an ethyl group, an isopropyl group, a tert-butyl group, a phenyl group, and a chlorine atom are preferable; a hydrogen atom, a methyl group, an isopropyl group, and a tert-butyl group are more preferable; and a hydrogen atom and a methyl group are even more preferable.

n is 0 or 1. n is preferably 1 from the viewpoint of solubility in an aliphatic polymer represented by polyolefins. In addition, n is preferably 1 from the viewpoint that then, a distance between an end portion of a modified polyolefin and a functional group being introduced is increased, and thereby reactivity of the modified polyolefin is increased.

Q is $-C(R^{18})(R^{19})-$, -C(=O)-, -S-, or $-S(=O)_2-$. Q is preferably $-C(R^{18})(R^{19})-$from the viewpoint of solubility in a resin, particularly a polyolefin, and from the viewpoint that solubility in a polyolefin is then increased during melt-kneading.

$R^{18}$ and $R^{19}$ are each independently a hydrogen atom, a hydrocarbon group having 1 to 6 carbon atoms, or a halogen atom, where $R^{18}$ and $R^{19}$ may be linked to form an aromatic ring or an aliphatic ring. Examples of hydrocarbon groups having 1 to 6 carbon atoms include a methyl group, an ethyl group, a propyl group, a butyl group, a hexyl group, a cyclohexyl group, a phenyl group, and the like. An example in which $R^{18}$ and $R^{19}$ are linked includes a 1,1-cyclohexylene group. Examples of halogen atoms include a fluorine atom, a chlorine atom, a bromine atom, an iodine atom, and the

like. For $R^{18}$ and $R^{19}$, a hydrogen atom, a methyl group, an ethyl group, and a phenyl group are preferable.

A in (iii) is the group represented by General Formula [V].

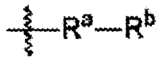

··· [V]

$R^a$ is an alkylene group having 1 to 5 carbon atoms or an arylene group having 6 to 10 carbon atoms. Examples of alkylene groups having 1 to 5 carbon atoms include a 1,1-methylene group, a 1,2-ethylene group, a 1,3-propylene group, a 1,4-butylene group, a 1,5-pentylene group, and the like. Examples of arylene groups having 6 to 10 carbon atoms include a phenylene group, a naphthylene group, and the like. For $R^a$, a 1,2-ethylene group, a 1,3-propylene group, a 1,4-butylene group, and a phenylene group are preferable. In the case of an alkylene group, a melting point of a nitrile oxide compound is likely to be increased as a carbon chain becomes shorter.

$R^b$ is a polar functional group. Examples of polar functional groups include a hydroxy group, a mercapto group, a carboxy group, an amino group, an amino group having a substituent, an amide group, an ether group, $-OR^{20}$ (where $R^{20}$ is an alkyl group or an aryl group), a heterocyclic ring, and the like. The heterocyclic ring is a cyclic substituent having a heteroatom such as a boron atom, a nitrogen atom, an oxygen atom, and a sulfur atom. Examples of heterocyclic rings include a furyl group, a thienyl group, a pyrrolyl group, an imidazolyl group, a pyranyl group, a pyridinyl group, a pyrimidinyl group, a pyrazinyl group, a pyrrolidinyl group, a piperidinyl group, a piperazinyl group, a morpholinyl group, a carbazolyl group, an imidazolidonyl group, and the like. The heterocyclic ring may have a substituent. $R^b$ is preferably a hydroxyl group, a mercapto group, a carboxy group, an amino group, an amino group having a substituent, or an amide group from the viewpoint that a melting point at room temperature or higher is then exhibited by a hydrogen bond. $R^b$ is preferably a heteroaryl ring from the viewpoint that then, a rigid skeleton is provided, and thereby a melting point at room temperature or higher is exhibited. A hydroxy group, a mercapto group, a carboxy group, an amino group, and an amino group having a substituent are particularly preferable.

In addition, $R^b$ is preferably a hydroxy group, an amino group, an amino group having a substituent, a mercapto group, a carboxyl group, and a heterocyclic ring are preferable from the viewpoint that reactivity with a filler or another resin is then high.

In the first aspect of the present invention, a melting point of the nitrile oxide compound is 25°C to 300°C, preferably 40°C to 280°C, more preferably 60°C to 260°C, and even more preferably 80°C to 240°C.

In the third aspect of the present invention, a melting point of the nitrile oxide compound is preferably 25°C to 300°C, more preferably 40°C to 280°C, even more preferably 60°C to 260°C, and particularly preferably 80°C to 240°C.

In a case where a melting point of the nitrile oxide compound is equal to or higher than the lower limit value of the above range, mobility at room temperature is reduced, and thereby storage stability at room temperature is improved. In a case where a melting point of the nitrile oxide compound is equal to or lower than the upper limit value of the above range, solubility in a resin at a reaction temperature is increased during a melting reaction with the resin, and thereby the reaction is facilitated. In a case where a melting point of the nitrile oxide compound is equal to or lower than the upper limit value of the above range, the nitrile oxide compound is easily melted during a melting reaction, and thereby reactivity thereof is increased.

In order to raise a melting point of the nitrile oxide compound to 25°C or higher, for example, a highly symmetric structure is added to A to increase symmetry of a molecular structure; or a highly rigid group, a short-chain group, or a group capable of hydrogen bonding is introduced into A.

An equivalent of nitrile oxide of the nitrile oxide compound of the present invention is 1.0 to 4.5 mmol/g, preferably 1.2 to 4.4 mmol/g, and more preferably 1.5 to 4.3 mmol/g. In a case where an equivalent of nitrile oxide of the nitrile oxide compound is equal to or more than the lower limit value of the above range, an amount of functional group per mass increases. In addition, a molecular weight of the nitrile oxide compound is suppressed to be low, and therefore problems of compatibility and a viscosity ratio hardly occur particularly in a reaction with a polymer. Accordingly, a reactivity of the nitrile oxide compound is increased. In a case where an equivalent of nitrile oxide of the nitrile oxide compound is equal to or less than the upper limit value of the above range, molecular weight movement is inhibited, and a side reaction such as intermolecular dimerization is inhibited.

Preferable specific examples of the nitrile oxide compound of the present invention include the following compounds. The letter "Ph" in the structural formula indicates a phenyl group.

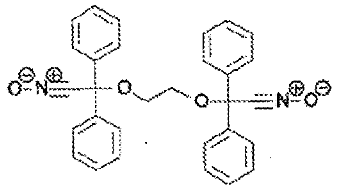

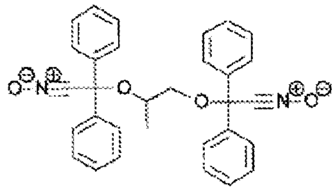

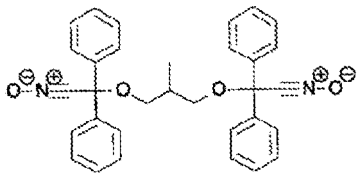

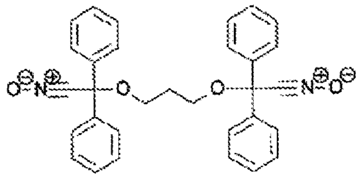

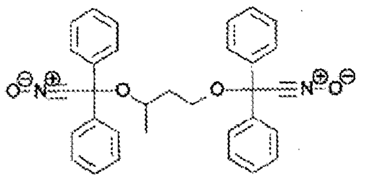

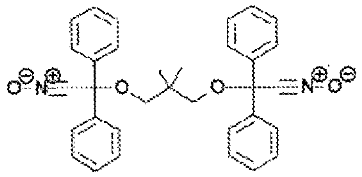

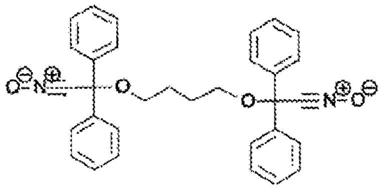

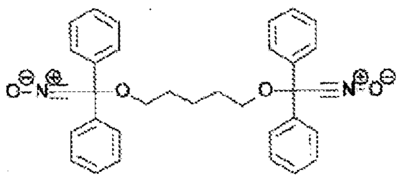

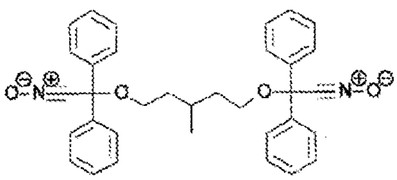

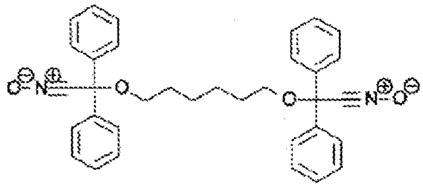

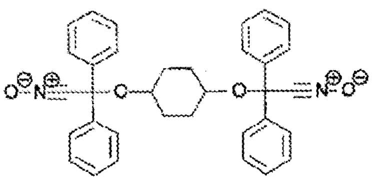

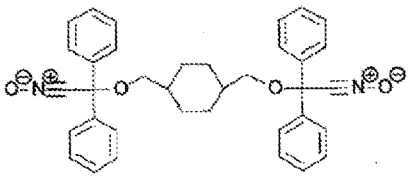

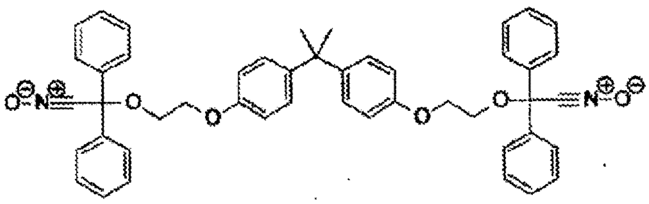

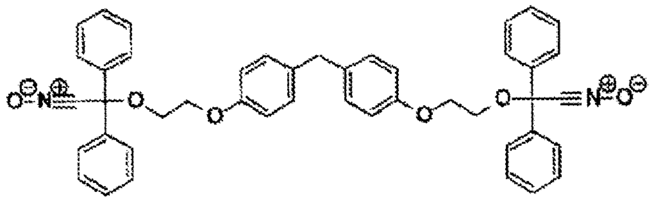

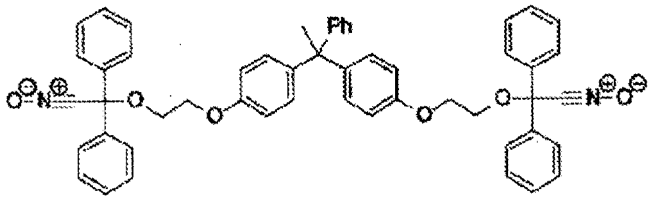

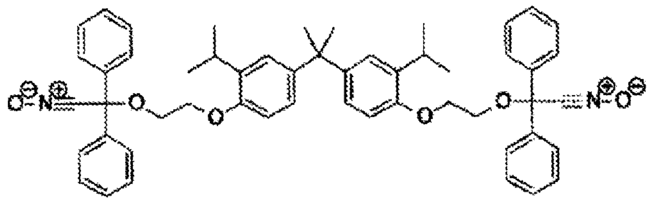

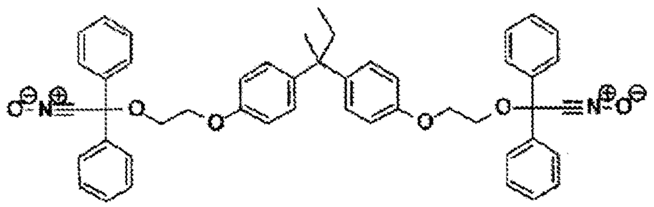

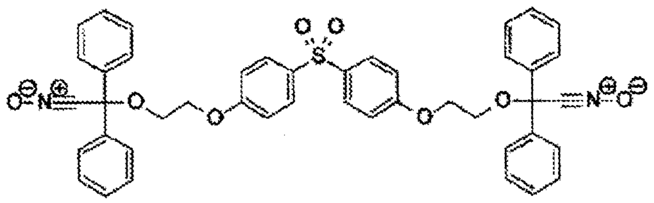

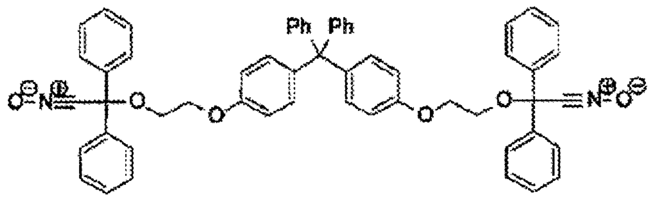

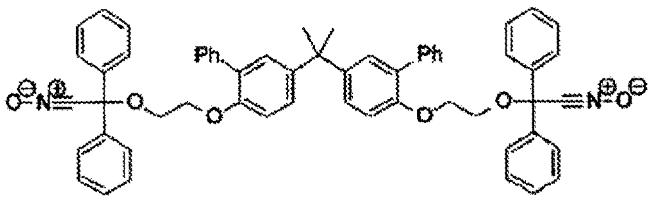

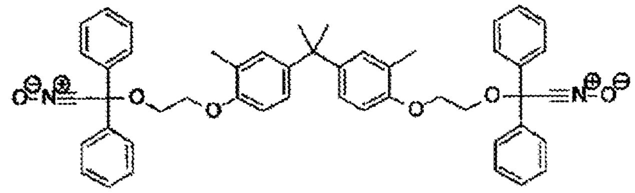

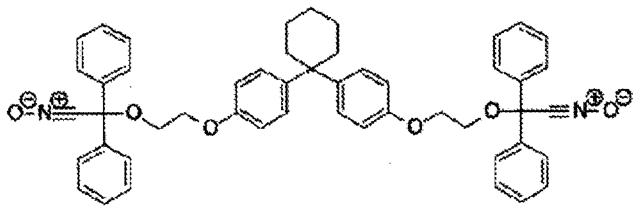

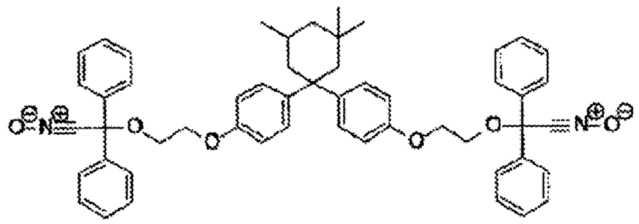

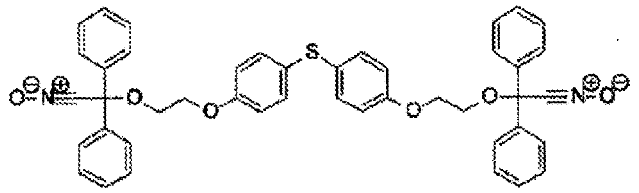

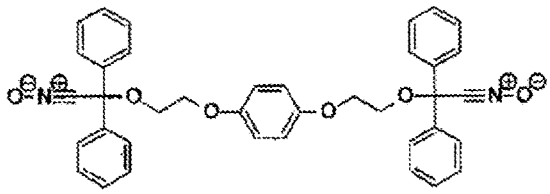

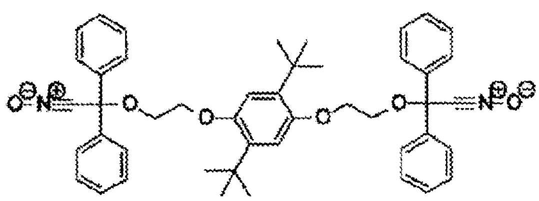

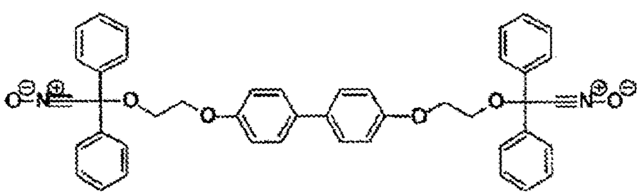

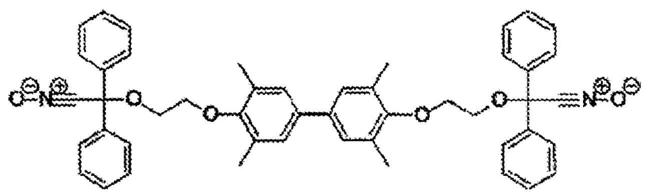

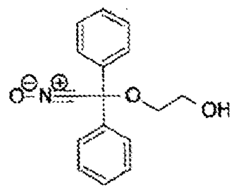

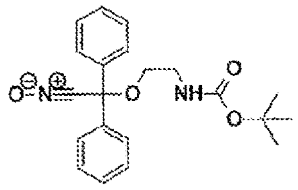

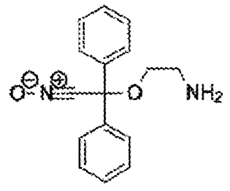

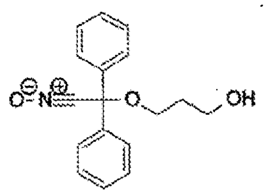

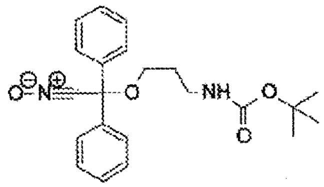

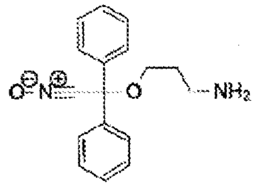

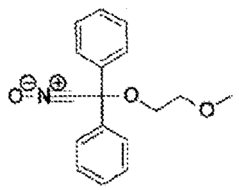

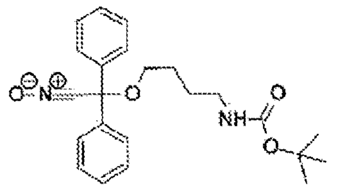

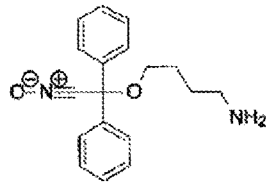

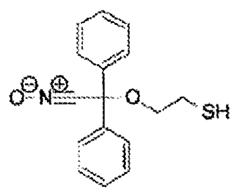

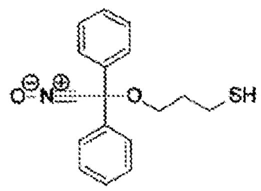

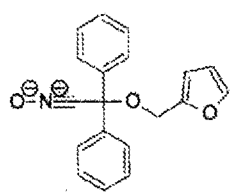

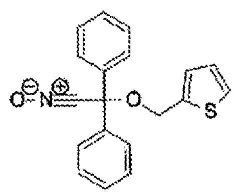

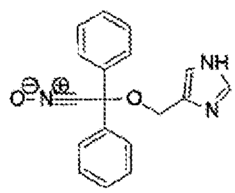

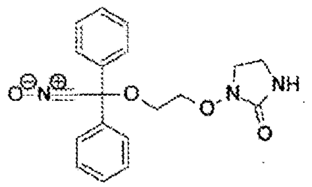

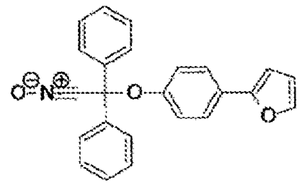

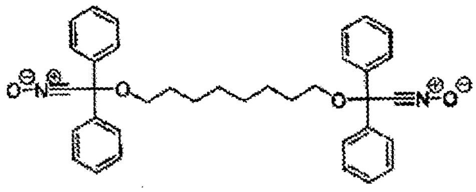

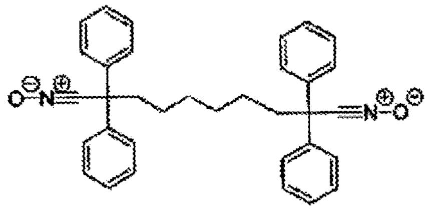

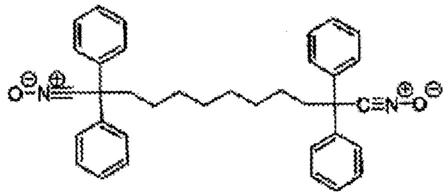

In addition to the compounds exemplified above, it is also possible to exemplify a structure in which at least one phenyl group at the α-position of a nitrile oxide group is substituted by a methylphenyl group or a dimethylphenyl group.

(Production of nitrile oxide compound)

The nitrile oxide compound can be produced, for example, according to the following scheme.

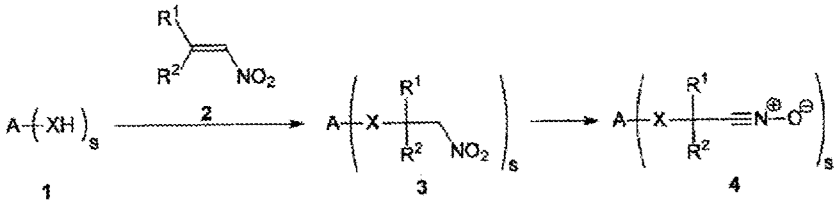

2

1 3 4

Compound 1 is reacted with a base such as sodium hydride, and then the resultant is reacted with Compound 2. Thereby, Compound 3 is obtained. Compound 3 is subjected to a dehydration reaction with phenyl isocyanate or the like, and thereby Nitrile Oxide Compound 4 is obtained.

Examples of Compound 1 include alcohols having 1 to 4 hydroxy groups, thiols having 1 to 4 mercapto groups, amines having 1 to 4 amino groups, and the like.

Examples of Compound 2 include 1-nitro-2,2-diphenylethylene and the like. By substituting a phenyl group of 1-nitro-2,2-diphenylethylene by another group, various substituents can be introduced into a carbon atom at the α-position of a nitrile oxide group.

(Mechanism of action)

The nitrile oxide compound of the present invention described above has three basic characteristics of (1) the structure represented by General Formula [I], (2) a melting point of 25°C to 300°C, and (3) an equivalent of nitrile oxide of 1.0 to 4.5 mmol/g. These three characteristics combine and result in the specificity of the present invention in which the compound is easily synthesized, has high reactivity, but still has excellent storage stability at room temperature, and has a nitrile oxide group that is hardly isomerized even at a high temperature.

The reason why the nitrile oxide compound of the present invention exhibits such effects is as follows.

- The nitrile oxide compound of the present invention can be easily synthesized by the above-described scheme since

-O-, -S-, or -N($R^3$)- is bonded to a carbon atom at the α-position of a nitrile oxide group.

- The heat stability of the nitrile oxide compound of the present invention can be enhanced by bonding of -$CH^2$- to a carbon atom at the α-position of a nitrile oxide group.
- The nitrile oxide compound of the present invention has high reactivity since an equivalent of nitrile oxide is 1.0 mmol/g or more.
- The nitrile oxide compound of the present invention has excellent storage stability at room temperature, because two sterically bulky substituents are introduced into a carbon atom at the α-position of a nitrile oxide group, and thereby dimerization is inhibited; and because molecular mobility during storage at room temperature is significantly reduced since a melting point is 25°C or higher to inhibit frequency of collisions between nitrile oxide groups, and thereby dimerization is inhibited.
- In the nitrile oxide compound of the present invention, a nitrile oxide group is hardly isomerized even at a high temperature, because a carbon atom at the α-position of the nitrile oxide group is an aliphatic carbon.

In addition, in a case of using, as a resin modifier or a rubber modifier, the nitrile oxide compound of the present invention which has high reactivity, but still has excellent storage stability at room temperature, and in which a nitrile oxide group is hardly isomerized even at a high temperature, it is possible to obtain a modified resin or a modified rubber while excellent reactivity is exhibited at a high modification temperature that is industrially advantageous.

It is also possible to solidify a polymer compound by introducing a nitrile oxide group into an end, main chain, or side chain of the polymer compound as in the case of the nitrile oxide compound disclosed in Patent Literature 2. However, in such a nitrile oxide compound, because a mass ratio of a polymer portion in a molecule is large, an equivalent of nitrile oxide is small, and reactivity is extremely low. In addition, in a case where such a nitrile oxide compound is used for modification or the like of a resin or diene rubber, a reaction between polymers occurs, leading to problems of compatibility and a viscosity ratio, and thereby reactivity is further lowered depending on reaction targets.

The present invention has noticeable differences from the inventions disclosed in the documents of the related art in constitutional requirements (specific matters of the invention) and effects of the invention. The present invention is particularly different from the findings of the related art, and not even a part of the results of the present invention, in which a solid nitrile oxide compound having two bulky substituents at the α-position of a nitrile oxide group exhibits excellent stability, has been gained from the documents of the related art. In fact, Patent Literature 1, Patent Literature 2, and Patent Literature 3 disclose that a reactant may be liquid or solid. In addition, Patent Literature 1, Patent Literature 2, and Patent Literature 3 do not disclose what molecular structure is necessary for exhibiting a high melting point.

<Composition>

A composition of the present invention contains the nitrile oxide compound of the present invention, and a substance that may react with a nitrile oxide group of the nitrile oxide compound of the present invention.

One kind or two or more kinds of the nitrile oxide compound of the present invention may be contained in the composition of the present invention.

One kind or two or more kinds of the substance that may react with a nitrile oxide group may be contained in the composition of the present invention.

If necessary, the composition of the present invention may contain a substance (a resin, a filler, an additive, and the like) which does not react with a nitrile oxide group as long as the effects of the present invention are not impaired.

(Substance that may react with nitrile oxide group)

The substance that may react with a nitrile oxide group is a substance having a group that may react with a nitrile oxide group.

Examples of groups that may react with a nitrile oxide group include a group having a double bond, an unsaturated bond group such as a group having a triple bond, and a highly nucleophilic group such as an amino group.

Examples of double bonds include a carbon-carbon double bond, a carbon-nitrogen double bond, a nitrogen-nitrogen double bond, a carbon-sulfur double bond, a carbon-phosphorus double bond, a carbon-arsenic double bond, a carbon-selenium double bond, a boron-nitrogen double bond, a nitrogen-phosphorus double bond, and the like.

Examples of triple bonds include a carbon-carbon triple bond, a carbon-nitrogen triple bond, a carbon-phosphorus triple bond, and the like.

The group that may react with a nitrile oxide group preferably has at least one bond selected from the group consisting of a carbon-carbon double bond, a carbon-carbon triple bond, a carbon-nitrogen double bond, and a carbon-nitrogen triple bond. Specific examples thereof include an alkenyl group, an alkynyl group, and a nitrile group.

Examples of the "substance" referred to in the phrase, the substance that may react with a nitrile oxide group, include an organic material (a resin, a rubber, a low molecular compound, and the like), an inorganic material (glass,

ceramic, metal, and the like), and the like. The substance is preferably an organic material and is more preferably a resin or rubber.

The substance that may react with a nitrile oxide group is preferably a polyolefin having a double bond, a diene rubber, or a polyacrylonitrile from the viewpoint that then, an industrially useful modified resin or modified rubber can be obtained.

Examples of polyolefins include polyethylene, polypropylene, and the like. Examples of double bonds of polyolefins include a vinyl group, a vinylidene group, a vinylene group, and the like.

Diene rubbers are rubbers produced using a diene monomer as a raw material. Examples of diene rubbers include a styrene-butadiene rubber, a nitrile rubber, a butadiene rubber, an isoprene rubber, a natural rubber, a chloroprene rubber, an acrylonitrile-butadiene rubber, a butyl rubber, an ethylene-propylene-diene rubber, and the like.

A content of the nitrile oxide compound in the composition is preferably 0.001 % to 10% by mass, more preferably 0.01% to 8% by mass, and even more preferably 0.05% to 6% by mass with respect to a total mass of the composition.

A content of the substance that may react with a nitrile oxide group is preferably 1% to 99.999% by mass and more preferably 20% to 99.9% by mass with respect to the total mass of the composition.

(Modified substance)

A modified substance (a modified resin, a modified rubber, and the like) is obtained by reacting the nitrile oxide compound of the present invention with the substance that may react with a nitrile oxide group, in the composition of the present invention.

It is possible to modify solubility characteristics, surface properties, and the like of a substance by modifying the substance. Specifically, a modified substance has improved solubility, wettability, adhesiveness, compatibility, and the like with respect to various materials as compared to a substance before being modified.

A modification reaction may be performed under air or under an inert atmosphere, and it is preferably performed under an inert atmosphere and more preferably under a nitrogen gas or an argon gas.

The modification reaction may be performed in the presence or absence of a solvent, and is preferably performed in the absence of a solvent from the viewpoint of productivity.

The solvent may be any solvent as long as the nitrile oxide compound of the present invention can be dissolved therein. Examples thereof include an aromatic solvent (toluene, xylene, and the like) and an aliphatic hydrocarbon solvent (hexane and the like).

In a case where the modification reaction is performed in the absence of a solvent, it is preferable to use a kneading device.

Examples of kneading devices include an extruder (a single-screw extruder, a twin-screw extruder, a multi-screw extruder, and the like), a kneader (a closed kneader, a Banbury mixer, and the like), and the like. The kneading device may be a continuous type or a batch type, and a continuous type is preferable from the viewpoint of production efficiency.

A modification temperature may be a temperature at which the nitrile oxide compound of the present invention reacts with the substance that may react with a nitrile oxide group. For example, a temperature is preferably as high as possible within a range at which the nitrile oxide compound is not decomposed from the viewpoint that, because the reaction is a chemical reaction, the reaction is accelerated if a temperature is high, and thereby production efficiency is increased. A modification temperature is preferably 0°C to 400°C, more preferably 50°C to 300°C, even more preferably 100°C to 250°C, and particularly preferably 160°C to 230°C.

(Mechanism of action)

Since the composition of the present invention described above contains the nitrile oxide compound of the present invention which has high reactivity and but still has excellent storage stability at room temperature, the composition has excellent storage stability at room temperature regardless of high reactivity of the nitrile oxide compound. In addition, since the composition contains the nitrile oxide compound of the present invention in which a nitrile oxide group is hardly isomerized even at a high temperature, the composition is excellent in reactivity between the nitrile oxide compound at a high temperature and the substance that may react with the nitrile oxide group of the nitrile oxide compound, without isomerization of the nitrile oxide group even at a high temperature.

<Method for producing modified polyolefin>

A method for producing a modified polyolefin of the present invention is a method including performing an addition reaction of a polyolefin having a carbon-carbon double bond at one end of two ends of a main chain (hereinafter, also referred to as a "one-end-double-bonded polyolefin") with a compound having a 1,3-dipolar functional group.

(One-end-double-bonded polyolefin)

Examples of the "polyolefin" referred to in the term, one-end-double-bonded polyolefin, include polyethylene, polypropylene, and the like.
Polyethylene may have more than 0 mol% and less than 50 mol% of an α-olefin other than ethylene as a comonomer. In addition, polyethylene may have more than 0 mol% and 1 mol% or less of a diene compound as a comonomer. A content of diene as a comonomer is preferably 0 mol%.
Polypropylene may have more than 0 mol% and less than 50 mol% of an α-olefin other than propylene as a comonomer. In addition, polyethylene may have more than 0 mol% and 1 mol% or less of a diene compound as a comonomer. A content of diene as a comonomer is preferably 0 mol%.
Examples of α-olefins include ethylene, propylene, butene, hexene, octene, 4-methyl-1-pentene, and the like.
Examples of diene compounds include vinyl norbornene, dicyclopentadiene, ethylidene norbornene, 1,4-hexadiene, and the like.
The one-end-double-bonded polyolefin has a carbon-carbon double bond at one end of two ends of a main chain. The one-end-double-bonded polyolefin may further have a carbon-carbon double bond in the middle of the main chain, or may further have a carbon-carbon double bond in the end or in the middle of the side chain.
Examples of carbon-carbon double bonds include a vinyl group, a vinylidene group, a vinylene group, and the like.
The carbon-carbon double bond is preferably a vinyl group or a vinylidene group, and is preferably a vinyl group from the viewpoint that reactivity with a 1,3-dipolar functional group is then excellent. 1,3-dipolar functional groups has higher reactivity with sterically small olefinic bonds than sterically large olefinic bonds.
The number of carbon-carbon double bonds per one molecule of the one-end-double-bonded polyolefin is preferably 0.1 to 4.0 bonds/molecule, more preferably 0.3 to 2.0 bonds/molecule, and even more preferably 0.5 to 1.5 bonds/molecule. In a case where the number of carbon-carbon double bonds per one molecule of the one-end-double-bonded polyolefin is equal to or more than the lower limit value of the above range, the number of polymer chains having an unsaturated bond at a termination end increases, and thereby functions are easily imparted to the polyolefin. In a case where the number of carbon-carbon double bonds per one molecule of the one-end-double-bonded polyolefin is equal to or less than the upper limit value of the above range, the number of polymer chains in which an unsaturated double bond is present only at one end of a polymer increases, and this is advantageous for filler composition and surface modification.
A mass average molecular weight (Mw) of the one-end-double-bonded polyolefin is preferably 10,000 to 1,000,000, more preferably 20,000 to 600,000, and even more preferably 30,000 to 500,000. In a case where a Mw of the one-end-double-bonded polyolefin is equal to or more than the lower limit value of the above range, strength is increased by entanglement of polymers. In a case where a Mw of the one-end-double-bonded polyolefin is equal to or less than the upper limit value of the above range, thermoforming is easy.
A molecular weight distribution (Mw/Mn) of the one-end-double-bonded polyolefin is preferably 1.1 to 6.0, more preferably 1.4 to 5.0, and even more preferably 1.6 to 4.0. In a case where a Mw/Mn of the one-end-double-bonded polyolefin is equal to or more than the lower limit value of the above range, fluidity during melting modification is increased, and thereby productivity can be increased. In a case where a Mw/Mn of the one-end-double-bonded polyolefin is equal to or less than the upper limit value of the above range, molecular weights of polymer chains are uniform, and thereby molecular design and prediction of physical properties are facilitated.
The one-end-double-bonded polyolefin can be generally produced by coordination polymerization of an α-olefin using a transition metal catalyst and not using a chain transfer agent.
Since a molecular weight is usually adjusted by a chain transfer agent in production of polyolefins, an end structure of a polyolefin is a saturated end. Meanwhile, in a case where a chain transfer agent such as hydrogen or organoaluminum is not used in coordination polymerization using a transition metal catalyst, a termination end of a polyolefin is a carbon-carbon double bond. In particular, an end double bond is likely to be generated in a polymerization system in which a molecular weight is adjusted by a polymerization temperature. Furthermore, in accordance with generation of hydrogen, a carbon-carbon double bond may be generated in the middle of a main chain and also in a side chain. These are disclosed in Macromolecules, Vol. 38, 2005, p. 6988-6996; Topics in Catalysis, Vol. 7, 1999, p. 145-163; and the like.
Examples of transition metal catalysts include complex catalyst systems (Phillips catalysts, metallocene catalysts, post-metallocene catalysts, and the like), Ziegler-Natta catalyst system, and the like. Among the examples, Phillips catalysts, metallocene catalysts, and post-metallocene catalysts are preferable, and from the viewpoint of a wide range of production, metallocene catalysts and post-metallocene catalysts are particularly preferable.
Furthermore, a small amount of a chain transfer agent may be added to a system if necessary.

(Compound having 1,3-dipolar functional group)

Examples of 1,3-dipolar functional groups include allyl anion type dipole functional groups (a nitron group, an azomethine imine group, an azomethine ylide group, an azine group, an azoxy group, a nitro group, a carbonyl ylide group, a carbonyl imine group, a carbonyl oxide group, a nitrosimine group, a nitrosoxide group, and the like), propargyl (or allenyl) anion type dipole functional groups (a nitrile oxide group, a nitrile imine group, a nitrile ylide group, a diazoalkane group, an azide group, and the like), and a combination of two or more thereof.

A 1,3-dipolar functional group is preferably a nitrile oxide group or a nitrone group, and is more preferably a nitrile oxide group from the viewpoint that then, a 1,3-dipolar cycloaddition reaction with an unsaturated bond proceeds in the absence of a catalyst.

The compound having a 1,3-dipolar functional group may have two or more 1,3-dipolar functional groups in a molecule.

The compound having a 1,3-dipolar functional group is preferably a nitrile oxide compound or a nitrone compound, and is more preferably a nitrile oxide compound from the viewpoint that then, an addition reaction with the one-end-double-bonded polyolefin proceeds in the absence of a catalyst.

Nitrile oxide compounds are classified into aromatic nitrile oxide compounds and aliphatic nitrile oxide compounds according to a carbon atom to which a nitrile oxide group is bonded. A nitrile oxide compound is preferably an aliphatic nitrile oxide compound from the viewpoint that a nitrile oxide group is then hardly isomerized. An aliphatic nitrile oxide compound is as described in the above <Nitrile oxide compound>. An aliphatic nitrile oxide compound preferably has the specific structure represented by General Formula [I], has a melting point of 25°C to 300°C, and has an equivalent of nitrile oxide of 1.0 to 4.5 mmol/g.

(Aromatic nitrile oxide compound)

Examples of aromatic nitrile oxide compounds include a compound represented by General Formula [VI] and a compound represented by General Formula [VII].

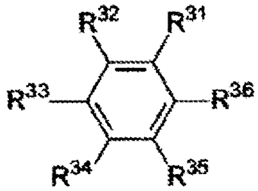

···[VI]

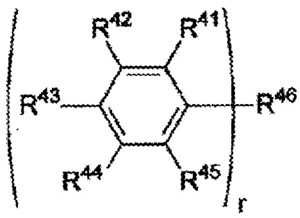

···[VII]

In General Formula [VI], any one or more of $R^{31}$ to $R^{36}$ are nitrile oxide groups. The residuals of $R^{31}$ to $R^{36}$ each independently represent a hydrogen atom, an alkyl group, an alkoxy group, an alicyclic hydrocarbon group, an aromatic hydrocarbon group, a heteroaryl group, a halogen atom, a hydroxy group, or an amino group. Two adjacent substituents may be bonded to each other to form a ring.

In General Formula [VII], any one or more of $R^{41}$ to $R^{45}$ are nitrile oxide groups. The residuals of $R^{41}$ to $R^{45}$ each independently represent a hydrogen atom, an alkyl group, an alkoxy group, an alicyclic hydrocarbon group, an aromatic hydrocarbon group, a heteroaryl group, a halogen atom, a hydroxy group, or an amino group. Two adjacent substituents may be bonded to each other to form a ring. $R^{46}$ is a spacer connecting aromatic rings, and is a divalent or higher valent organic group having 1 to 20 carbon atoms or an oxygen atom. r is an integer of 2 or more.

(Production of modified polyolefin)

The modified polyolefin can be obtained by, for example, mixing the one-end-double-bonded polyolefin with the compound having a 1,3-dipolar functional group, and a subjecting the mixture to a heat treatment.

One kind of the one-end-double-bonded polyolefin may be used, or two or more kinds thereof may be used in

combination. One kind of the compound having a 1,3-dipolar functional group may be used, or two or more kinds thereof may be used in combination.

A blending amount of the compound having a 1,3-dipolar functional group is preferably 0.001 to 10 parts by mass, more preferably 0.01 to 8 parts by mass, and even more preferably 0.05 to 6 parts by mass with respect to 100 parts by mass of the one-end-double-bonded polyolefin.

The heat treatment may be performed in a solvent, or may be performed while performing melt-kneading under conditions substantially free of a solvent. The heat treatment is preferably performed while performing melt-kneading from the viewpoint of productivity.

Examples of solvents include hydrocarbons (toluene, xylene, hexane, cyclohexane, and the like) and the like.

Examples of methods of melt-kneading include a method using a known device such as a twin-screw extruder or a Banbury mixer. Details of the method of melt-kneading are disclosed in, for example, "Thermoplastic Elastomers 2nd. ed.," Hanser Gardner Publications, 1996, p. 153-190.

In a case of performing melt-kneading, all of components may be melt-kneaded at once, or some of components may be melt-kneaded, and then the remaining components may be additionally melt-kneaded. The melt-kneading may be performed twice or more. After the melt-kneading, an additional heat treatment may be performed.

In a case where a solvent is used, a modification temperature is preferably 50°C to 200°C, more preferably 60°C to 180°C, and even more preferably 80°C to 150°C. In a case where a solvent is used, a modification time is preferably 30 minutes to 24 hours, more preferably 1 to 15 hours, and even more preferably 2 to 10 hours.

In a case of substantially free of a solvent, that is, in a case where melt-kneading is performed, a modification temperature is preferably 80°C to 300°C, more preferably 120°C to 280°C, and even more preferably 160°C to 260°C. In a case of performing the melt-kneading, a modification time is preferably 1 second to 30 minutes and more preferably 10 seconds to 5 minutes.

In a case where the one-end-double-bonded polyolefin is a one-end-double-bonded polypropylene, and the compound having a 1,3-dipolar functional group is a nitrile oxide compound, a modified polypropylene is obtained as follows, for example.

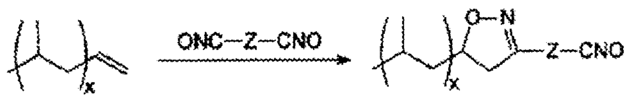


Z is -C($R^1$)($R^2$)-X-A-X-C($R^1$)($R^2$)-, CNO is a nitrile oxide group, and x is the number of propylene units in the polypropylene.

In a case where the one-end-double-bonded polyolefin is a one-end-double-bonded ethylene-propylene copolymer, and the compound having a 1,3-dipolar functional group is a nitrile oxide compound, a modified ethylene-propylene copolymer is obtained as follows, for example.

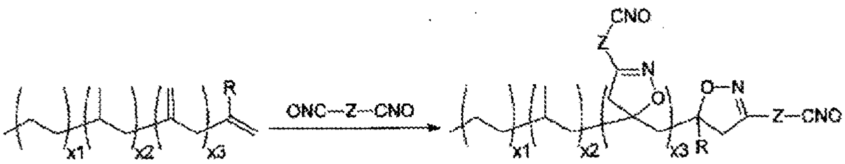


Z is -C($R^1$)($R^2$)-X-A-X-C($R^1$)($R^2$)-, CNO is a nitrile oxide group, R is a hydrogen atom or a methyl group, and x1 to x3 are each independently the number of each of units in the ethylene-propylene copolymer.

(Mechanism of action)

In the above-described method for producing a modified polyolefin of the present invention, since the polyolefin having a carbon-carbon double bond at one end of two ends of a main chain is used, it is possible to modify the polyolefin with the compound having a 1,3-dipolar functional group under industrially advantageous high-temperature conditions.

<Modified polyolefin>

The modified polyolefin of the present invention is obtained by the method for producing a modified polyolefin of the present invention.

The modified polyolefin of the present invention is preferably a modified polyolefin having a structure represented

by General Formula [VIII] from the viewpoint that then, a nitrile oxide group of a nitrile oxide compound used for modification is hardly dimerized and isomerized.

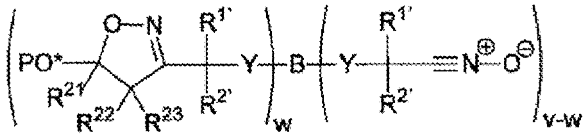
... [VIII]

v is an integer of 1 to 4. v is preferably an integer of 1 to 3 and more preferably 1 or 2 from the viewpoint that an increase in viscosity of the modified polyolefin is then suppressed.

w is an integer of 1 to 4. w is preferably an integer of 1 to 3 and more preferably 1 or 2 from the viewpoint that then, a block copolymer is produced by use as a filler dispersant or by reaction with another resin.

Where $v \geq w$.

PO* is a main chain of a polyolefin. Examples of polyolefins include the same examples as those exemplified for the "polyolefin" referred to in the one-end-double-bonded polyolefin, and preferable aspects thereof are also the same.

$R^{21}$, $R^{22}$, and $R^{23}$ are each independently a hydrogen atom or an alkyl group having 1 to 8 carbon atoms. Examples of alkyl groups having 1 to 8 carbon atoms include a methyl group, an ethyl group, an isopropyl group, an n-propyl group, an n-butyl group, an n-hexyl group, and the like. $R^{21}$ is preferably a hydrogen atom, a methyl group, an ethyl group, an n-butyl group, or an n-hexyl group. $R^{22}$ and $R^{23}$ are preferably a hydrogen atom or a methyl group, and more preferably a hydrogen atom, from the viewpoint that then, steric hindrance around a double bond is reduced, and reactivity is increased.

$R^{1'}$ and $R^{2'}$ are each independently a hydrocarbon group having 4 to 10 carbon atoms or a halogenated hydrocarbon group having 4 to 10 carbon atoms. Examples of $R^{1'}$ and $R^{2'}$ include the same examples as those exemplified for $R^1$ and $R^2$ in General Formula [I], and preferable aspects thereof are also the same.

Y's are each independently a divalent hydrocarbon group, -O-, -S-, or -N($R^{3'}$)-, where $R^{3'}$ is a hydrogen atom or a hydrocarbon group having 1 to 6 carbon atoms. Examples of Y and $R^{3'}$ include the same examples as those exemplified for X and $R^3$ in General Formula [I], and preferable aspects thereof are also the same.

B is a v-valent organic group. Examples of B include the same examples those exemplified for A in General Formula [I], and preferable aspects thereof are also the same.

For the modified polyolefin represented by General Formula [VIII], in a case where $^1$H-NMR is measured at 120°C using o-dichlorobenzene-d2 as a solvent, a signal derived from hydrogen atoms of $R^{21}$, $R^{22}$, and $R^{23}$ is observed within a range of 4.1 to 4.8 ppm and a range of 2.3 to 3.0 ppm.

(Composition)

The modified polyolefin of the present invention may be blended in with other components (a resin, a filler, an additive, and the like) to be formed into a composition.

Other components may be blended in before modification of the one-end-double-bonded polyolefin, may be blended in during the modification, or may be blended in after the modification.

A blending amount of other components is usually 0.001 to 200 parts by mass with respect to 100 parts by mass of the modified polyolefin.

Examples of fillers include inorganic fillers (talc, calcium carbonate, calcined kaolin, and the like) and organic fillers (fibers, wood powders, cellulose powders, and the like).

Examples of additives include antioxidants (phenol-based, sulfur-based, phosphorus-based, lactone-based, vitamin-based, and the like), weathering stabilizers, ultraviolet absorbents (benzotriazole-based, tridiamine-based, anilide-based, benzophenone-based, and the like), heat stabilizers, light stabilizers (hindered amine-based, benzoate-based, and the like), antistatic agents, nucleating agents, pigments, adsorbents (metal oxides such as zinc oxide and magnesium oxide), metal chlorides (iron chloride, calcium chloride, and the like), hydrotalcite, aluminate, lubricants (fatty acids, higher alcohols, fatty acid amides, fatty acid esters, and the like), mineral oils, silicone compounds, and the like.

(Mechanism of action)

In the modified polyolefin of the present invention described above, since one end of two ends of a main chain of the polyolefin is modified with the compound having a 1,3-dipolar functional group, in a case where the modified polyolefin is reacted with a polymer having a functional group that may react with a functional group of the modified polyolefin, it is possible to easily produce a block copolymer having excellent mechanical properties and the like.

In addition, in the modified polyolefin of the present invention, since one end of the main chain is modified with

the compound having a 1,3-dipolar functional group, a cross-linking reaction hardly occurs in a case of producing a block copolymer, and thereby the block copolymer is hardly cured. On the other hand, in a modified polyolefin in which both ends of a main chain are modified with the compound having a 1,3-dipolar functional group, a cross-linking reaction easily occurs in a case of producing a block copolymer, and thereby the block copolymer is easily cured.

Furthermore, in the modified polyolefin of the present invention, since one end of the main chain is modified with the compound having a 1,3-dipolar functional group, in a case where the modified polyolefin is introduced into a surface of a filler, the main chain of the polyolefin is aligned perpendicular to the surface of the filler. Accordingly, a density of the polyolefin on the surface of the filler increases, and physical properties by the polyolefin are likely to be exhibited. On the other hand, in the modified polyolefin in which both ends of the main chain are modified with the compound having a 1,3-dipolar functional group, in a case where the modified polyolefin is introduced into a surface of a filler, the main chain of the polyolefin is introduced into the surface of the filler along the surface. Accordingly, a density of the polyolefin on the surface of the filler decreases, and physical properties by the polyolefin are unlikely to be exhibited.

<Method for producing block copolymer>

A method for producing a block copolymer of the present invention is a method of reacting the modified polyolefin of the present invention with a polymer having a functional group that may react with a functional group of the modified polyolefin (hereinafter also referred to as a "reactive-functional-group-containing polymer").

One kind of the modified polyolefin may be used, or two or more kinds thereof may be used in combination. One kind of the reactive-functional-group-containing polymer may be used, or two or more kinds thereof may be used in combination.

Examples of functional groups of the modified polyolefin include groups introduced in a case where the one-end-double-bonded polyolefin is modified with the compound having a 1,3-dipolar functional group. Examples of groups include a 1,3-dipolar functional group, a hydroxy group, an amino group, an amino group having a substituent, a mercapto group, a carboxyl group, a heterocyclic ring, and the like. The functional group of the modified polyolefin is preferably a 1,3-dipolar functional group, a hydroxy group, an amino group, or an amino group having a substituent from the viewpoint that then, reactivity with a surface of a filler and a functional group of a different resin is high.

Examples of functional groups of the reactive-functional-group-containing polymer which may react with the functional group of the modified polyolefin include a carbon-carbon double bond, a carbon-carbon triple bond, a carbon-nitrogen triple bond, an acid anhydride group, an amino group, and the like.

Examples of reactive-functional-group-containing polymers include a polyolefin having a carbon-carbon double bond, a styrene-butadiene rubber, a nitrile rubber, a butadiene rubber, an isoprene rubber, a natural rubber, a chloroprene rubber, an acrylonitrile-butadiene rubber, a butyl rubber, an ethylene-propylene-diene rubber, polyacrylonitrile, and the like.

The block copolymer is obtained by, for example, mixing a modified polyolefin with a reactive-functional-group-containing polymer, and subjecting the mixture to a heat treatment.

A blending amount of the reactive-functional-group-containing polymer is preferably 10 to 900 parts by mass with respect to 100 parts by mass of the modified polyolefin.

The heat treatment may be performed in a solvent, or may be performed while performing melt-kneading under conditions substantially free of a solvent. The heat treatment is preferably performed while performing melt-kneading from the viewpoint of productivity.

Examples of solvents include hydrocarbons (toluene, xylene, hexane, cyclohexane, and the like) and the like.

Examples of methods of melt-kneading include a method using a known device such as a twin-screw extruder or a Banbury mixer. Details of the method of melt-kneading are disclosed in, for example, "Thermoplastic Elastomers 2nd. ed.," Hanser Gardner Publications, 1996, p. 153-190.

In a case of performing melt-kneading, all of components may be melt-kneaded at once, or some of components may be melt-kneaded, and then the remaining components may be additionally melt-kneaded. The melt-kneading may be performed twice or more. After the melt-kneading, an additional heat treatment may be performed.

In a case where a solvent is used, a reaction temperature is preferably 50°C to 200°C, more preferably 60°C to 180°C, and even more preferably 80°C to 150°C. In a case where a solvent is used, a reaction time is preferably 30 minutes to 24 hours, more preferably 1 to 15 hours, and even more preferably 2 to 10 hours.

In a case of substantially free of a solvent, that is, in a case where melt-kneading is performed, a reaction temperature is preferably 80°C to 300°C, more preferably 120°C to 280°C, and even more preferably 160°C to 260°C. In a case of performing the melt-kneading, a reaction time is preferably 1 second to 30 minutes and more preferably 10 seconds to 5 minutes.

In a case where the modified polyolefin is a modified polypropylene, and the reactive-functional-group-containing polymer is an ethylene-propylene-dicyclopentadiene rubber, a block copolymer is obtained as follows, for example.

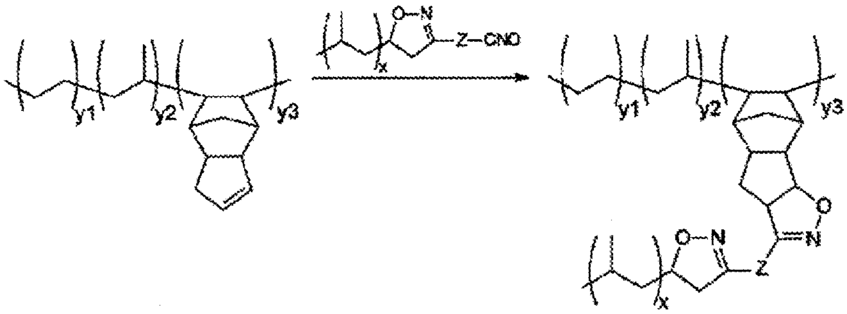

Z is -C($R^1$)($R^2$)-X-A-X-C($R^1$)($R^2$)-, CNO is a nitrile oxide group, x is the number of propylene units in the polypropylene, and y1 to y3 are each independently the number of each of units in the ethylene-propylene-dicyclopentadiene rubber.

In a case where the modified polyolefin is a modified polypropylene, and the reactive-functional-group-containing polymer is an ethylene-propylene copolymer, a block copolymer is obtained as follows, for example.

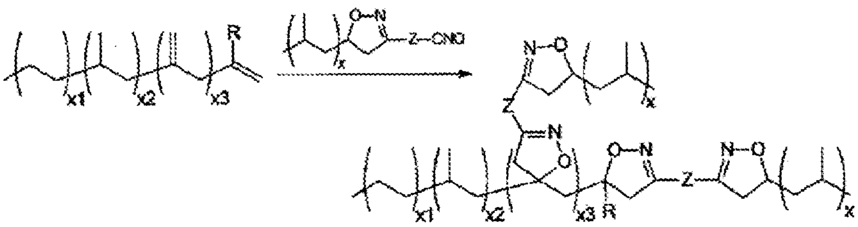

Z is -C($R^1$)($R^2$)-X-A-X-C($R^1$)($R^2$)-, CNO is a nitrile oxide group, R is a hydrogen atom or a methyl group, x is the number of propylene units in the polypropylene, and x1 to x3 are each independently the number of each of units in the ethylene-propylene copolymer.

(Composition)

The block copolymer may be blended in with other components (a resin, a filler, an additive, and the like) to be formed into a composition.

Other components may be blended in before reaction of the modified polyolefin with the reactive-functional-group-containing polymer, may be blended in during the reaction, or may be blended in after the reaction.

A blending amount of other components is usually 0.001 to 200 parts by mass with respect to 100 parts by mass of the block copolymer.

Examples of fillers and additives include the same examples as those exemplified for the above-described fillers and additives.

(Mechanism of action)

In the method for producing a block copolymer of the present invention described above, since the polyolefin in which one end of two ends of the main chain of the polyolefin has been modified with the compound having a 1,3-dipolar functional group is used, in a case where the modified polyolefin is reacted with the reactive-functional-group-containing polymer, it is possible to easily produce a block copolymer having excellent mechanical properties and the like.

[Examples]

Hereinafter, the present invention will be described more specifically with reference to examples, but the present invention is not limited to the following examples as long as the gist of the present invention is not exceeded.

<Measurement of physical properties, analysis, and the like>

(Melting point)

A temperature of a nitrile oxide compound was raised at a condition of 1°C/min using a melting point measuring device (manufactured by Stuart Scientific, SMP3), and a temperature at a time point when all solid parts were melted was defined as a melting point of the nitrile oxide compound. A melting point of nitrile oxide compounds that are liquid at 25°C was defined to be lower than 25°C.

(Equivalent of nitrile oxide)

An equivalent of nitrile oxide was obtained from the following equation.

$$\text{Equivalent of nitrile oxide [mmol/g]} = 1000 \times (\text{number of nitrile oxide groups in molecule/molecular weight of nitrile oxide compound})$$

(GPC measurement)

A mass average molecular weight (Mw), a number average molecular weight (Mn), and a molecular weight distribution (Mw/Mn) of a polyolefin were obtained by GPC measurement. The GPC measurement was performed using Alliance GPCV2000 manufactured by Waters. A differential refractometer was used as a detector, and four columns of TSKgel GMH6-HT manufactured by TOSOH CORPORATION were used as columns. Ortho-dichlorobenzene was used as a mobile phase solvent, and the solvent was flowed at 135°C and 1.0 mL/min. As standard samples, monodisperse polystyrene manufactured by Polymer Laboratories was used. From a viscosity equation of the polystyrene standard samples and the polyolefin, a calibration curve relating to a retention time and a molecular weight was created, and a molecular weight was calculated based on the calibration curve. Ten polystyrene standard samples having a molecular weight of 2,783,000, 1,412,000, 590,500, 164,500, 72,450, 29,510, 10,730, 3,950, 1,530, and 580 were used. As the viscosity equation, $[\eta] = K \times M\alpha$ was used. Regarding polystyrene, $K = 1.38 \times 10^{-4}$ and $\alpha = 0.70$ were adopted. Regarding polyethylene, $K = 4.77 \times 10^{-4}$ and $\alpha = 0.70$ were adopted. Regarding polypropylene, $K = 1.03 \times 10^{-4}$ and $\alpha = 0.78$ were adopted.

(DSC measurement)

A melting point (Tm) of a polymer was obtained by DSC measurement. For the DSC measurement, Diamond DSC manufactured by PerkinElmer Inc. was used.

After isothermal holding at 20°C for 1 minute, raising a temperature from 20°C to 210°C at 10°C/min, isothermal holding at 210°C for 5 minutes, lowering the temperature from 210°C to -70°C at 10°C/min, isothermal holding at -70°C for 5 minutes, and raising the temperature from -70°C to 210°C at 10°C/min, a melting point of a sample was measured.

(NMR measurement)

A ratio of each of units and the number of carbon-carbon double bonds in the polymer were obtained by NMR measurement. As a solvent, a mixed solvent of 1,2-dichlorobenzene/deuterated bromobenzene (4/1) to which a small amount of hexamethyldisiloxane was added as a reference substance was used. A concentration of sample was about 200 mg/2.4 mL. After the sample and the solvent were introduced into an NMR sample tube and sufficiently purged with nitrogen, the sample was dissolved in the solvent in a 130°C heat block to obtain a uniform solution. The measurement was carried out at 120°C using Bruker AVANCE III Cryo-NMR, 10 mmφ CryoProbe. Measurement conditions were as follows: for $^1$H-NMR, a solvent presaturation method, 4.5° pulse, a pulse interval of 2 seconds, and integration of 512 times; and for $^{13}$C-NMR, full proton decoupling conditions, a 90° pulse interval of 20 seconds for polyethylene and 15 seconds for polypropylene, and integration of 512 times.

As a reference peak at a chemical shift, a signal of a methyl group of hexamethyldisiloxane added to the measurement solvent was used. A peak at 0.08 ppm was used for $^1$H-NMR, and a peak at 1.979 ppm was used for $^{13}$C-NMR.

(Ratio of each of units in polymer)

A ratio of each of units in the polymer was obtained according to a method disclosed in Journal of Polymer Science Part A: Polymer Chemistry, Vol. 42, 2004, p. 2474-2482.

(Number of carbon-carbon double bonds in polymer)

The number of carbon-carbon double bonds per 1 mol of monomer unit [mol/mol-monomer unit] was obtained from the following formulas.

Number of end vinyl groups $\{(I_{5.05\text{ to }4.88} + I_{5.85\text{ to }5.70})/3\}/A$
Number of vinylidene groups $\{(I_{4.84\text{ to }4\cdot44})/2\}/A$
Number of vinylene groups $\{I_{5.52\text{ to }5.30}/2\}/A$
Number of trisubstituted olefins $\{I_{5.30\text{ to }5.05}\}/A$

I is an integrated intensity of a proton signal in $^{1}$H-NMR. For example, $I_{5.30\text{ to }5.05}$ indicates an integrated intensity of a proton signal detected between 5.30 ppm and 5.05 ppm in $^{1}$H-NMR. A was obtained from the following equation.

$$A = I_{3.00\text{ to }0.09}/\{4 \times (1 - C3 - C6) + 6 \times C3 + 12 \times C6\}$$

C3 is a ratio (0 to 1) of propylene units in the polymer, and C6 is a ratio (0 to 1) of hexene units in the polymer. For example, in a case of a propylene homopolymer, C3 = 1 and C6 = 0, and in a case of an ethylene-hexene copolymer, C3 = 0.

The number of carbon-carbon double bonds per 1 g of polyolefin is obtained from the following equation.

$$\text{Number of carbon-carbon double bonds per 1 g of polyolefin [mol/g]} = \text{number of carbon-carbon double bonds per 1 mol of monomer unit [mol/mol-monomer unit]}/\{28 \times (1 - C3 - C6) + 42 \times C3 + 84 \times C6\}$$

The number of carbon-carbon double bonds per one polyolefin molecule was obtained from the following equation.

$$\text{Number of carbon-carbon double bonds per one polyolefin molecule [bonds/molecule]} = \text{number of carbon-carbon double bonds per 1 g of polyolefin [mol/g]}/\text{number average molecular weight of polyolefin [g/mol]}$$

(Melt flow rate (MFR))

A measured value for polyethylene at a test temperature of 190°C and a nominal load of 2.16 kg (21.17 N) was shown as a MFR according to Condition D in Table 1 of Annex A of JIS K 7210: 2004.

A measured value for polypropylene at a test temperature of 230°C and a nominal load of 2.16 kg (21.17 N) was shown as a MFR according to Condition M in Table 1 of Annex A of JIS K 7210: 2004.

(Quantification of reaction inversion rate)

30 mg of modified polyolefins obtained in Examples 1B to 32B was dissolved in 0.7 mL of o-dichlorobenzene-$d_2$, and $^{1}$H-NMR was measured at 120°C. A reaction inversion rate was obtained from a ratio of a signal (2H) at 4.9 ppm which was derived from a vinyl group or a signal (2H) at about 4.7 ppm which was derived from a vinylidene group, to

a signal (1H) at about 4.4 to 4.6 ppm which was derived from an isoxazoline ring derived from a vinyl group or a signal (2H) at about 2.6 ppm which was derived from an isoxazoline ring derived from vinylidene.

(Tensile test)

Kneaded products obtained in Examples 33B and 34B and Comparative Examples 1B and 2B were pressed at 190°C for 2 minutes, and thereby sheets having a thickness of about 0.1 mm were produced.

A sheet was punched out with a dumbbell-shaped 6 (JIS K 6251) to produce a sample. Thereafter, a tensile test was performed at a test speed of 100 mm/min at 23°C to obtain a yield stress, a fracture stress, and a fracture strain.

In addition, after the sheet was punched out to produce a 10 mm wide strip, a tensile elastic modulus was measured at a test speed of 1 mm/min.

<Synthesis of compounds>

(Synthesis Example 1A)

Synthesis of 1-nitro-2,2-diphenylethylene:

1-Nitro-2,2-diphenylethylene was synthesized according to a method disclosed in Chemical Communications, Vol. 49, 2013, p. 7723-7725.

(Example 1A)

Synthesis of nitrile oxide compound A:

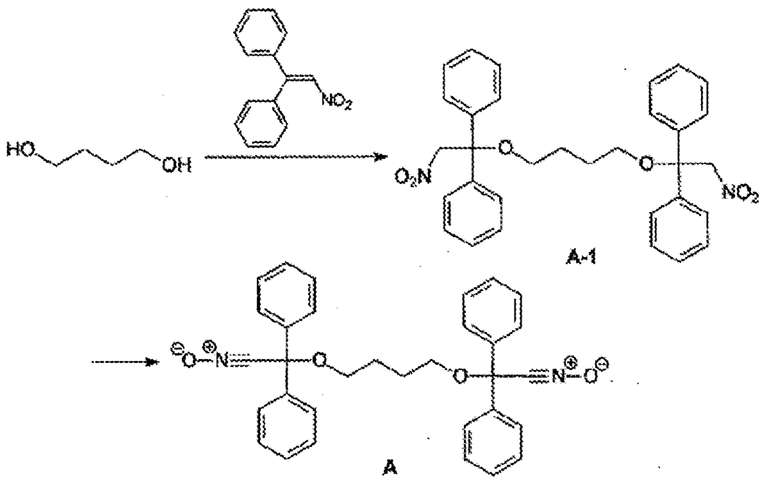

5.52 g (61.3 mmol) of 1,4-butanediol was dissolved in 300 mL of dehydrated tetrahydrofuran (THF) and cooled to 0°C. To this solution, 8 g (200 mmol) of sodium hydride was added under nitrogen gas, and the mixture was stirred at 0°C for 1 hour. To this solution, 30 g (133 mmol) of 1-nitro-2,2-diphenylethylene was added, and the mixture was stirred at 20°C for 16 hours. The solution was cooled to 0°C, and thereafter was neutralized with an aqueous solution of 2 mol/L hydrogen chloride until a pH reached 6 to 7. The neutralized solution was extracted using dichloromethane. The dichloromethane solution was washed with saturated saline, dried over anhydrous sodium sulfate, and filtered. The filtrate was concentrated and purified. The obtained solid was washed with a solution of ethyl acetate/petroleum ether = 1/1, and thereby 29 g of a compound A-1 as a white solid was obtained (yield: 88%).

NMR spectrum of compound A-1:

$^1$H-NMR (400 MHz, $CDCl_3$): δ7.38-7.22 (m, 20 H), 5.34 (s, 3 H), 5.39-5.30 (m, 1 H), 5.39-5.30 (m, 1 H), 3.40 (brs, 4 H), 1.79 (brs, 4 H) ppm.

29 g (53.6 mmol) of the compound A-1 was dissolved in 900 mL of dehydrated dichloromethane. To this solution,

33 g (215 mmol) of 4-chlorophenylisocyanate, 32.7 g (323 mmol) of triethylamine, and 60 g of molecular sieves 4A were added, and the mixture was stirred at 20°C for 16 hours under nitrogen gas. The solution was filtered. The filtrate was concentrated and then purified by column chromatography (acidic silica gel, solvent: n-hexane/ethyl acetate 1/0, 1/1). Thereby, 6.57 g of a nitrile oxide compound A as a white solid was obtained (yield: 24%). Table 1 shows a melting point and an equivalent of nitrile oxide of the nitrile oxide compound A.

NMR spectrum of nitrile oxide compound A:

$^1$ H-NMR (400 MHz, $CDCl_3$): δ7.47-7.29 (m, 20 H), 3.49 (brs, 4 H), 1.82 (brs, 4 H) ppm.
Melting point of nitrile oxide compound A: 135°C

(Example 2A)

Synthesis of nitrile oxide compound B:

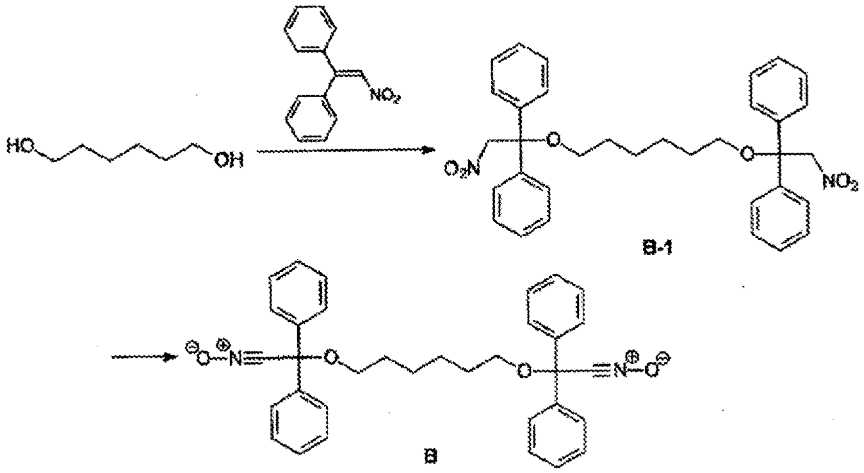

B-1

B

14.17 g (120 mmol) of 1,6-hexanediol was dissolved in 120 mL of dehydrated THF and cooled to 0°C. To this solution, 16 g (400 mmol) of sodium hydride was added under nitrogen gas, and the mixture was stirred at 0°C for 1 hour. To this solution, 60 g (266 mmol) of 1-nitro-2,2-diphenylethylene was added, and the mixture was stirred at 20°C for 16 hours. The solution was cooled to 0°C, and thereafter was neutralized with an aqueous solution of 2 mol/L hydrogen chloride until a pH reached 6 to 7. The neutralized solution was extracted using dichloromethane. The dichloromethane solution was washed with saturated saline, dried over anhydrous sodium sulfate, and filtered. The filtrate was concentrated and purified. The obtained solid was washed with ethyl acetate, and thereby 50 g of a compound B-1 as a white solid was obtained (yield: 70%).

NMR spectrum of compound B-1:

$^1$ H-NMR (400 MHz, $CDCl_3$): δ7.36-7.24 (m, 20 H), 5.34 (s, 4 H), 3.35 (t, 4 H), 1.74-1.61 (m, 4 H), 1.47-1.32 (m, 4 H) ppm.
25 g (44.0 mmol) of the compound B-1 was dissolved in 750 mL of dehydrated dichloromethane. To this solution, 22.5 mL (176 mmol) of 4-chlorophenylisocyanate, 26.90 g (266 mmol) of triethylamine, and 50 g of molecular sieves 4A were added, and the mixture was stirred at 20°C for 16 hours under nitrogen gas. The solution was filtered. The filtrate was concentrated and then purified by column chromatography (acidic silica gel, solvent: n-hexane/ethyl acetate 1/0, 3/1). Thereby, 7.87 g of a nitrile oxide compound B as a white solid was obtained (yield: 34%). Table 1 shows a melting point and an equivalent of nitrile oxide of the nitrile oxide compound B.

NMR spectrum of nitrile oxide compound B:

$^1$H-NMR (400 MHz, $CDCl_3$): δ7.44-7.30 (m, 20 H), 3.45 (t, 4 H), 1.69 (m4 H), 1.41 (m, 4 H) ppm.
Melting point of nitrile oxide compound B: 95°C

(Example 3A)

Synthesis of nitrile oxide compound C:

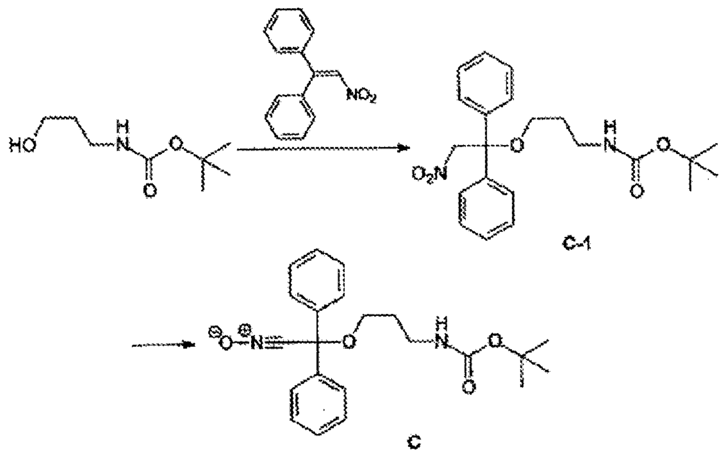

C-1

C

150 mg (3.4 mmol) of sodium hydride was washed three times with hexane, a gas phase was replaced with an argon gas. Thereafter, 10 mL of N,N-dimethylformamide (DMF) was added. To this solution, a DMF solution (10 mL) of 500 mg (2.8 mmol) of 4-(tert-butoxycarbonylamino)-1-propanol was added at 0°C, and the mixture was stirred for 2 hours. To this solution, a DMF solution (10 mL) of 430 mg (1.9 mmol) of 1-nitro-2,2-diphenylethylene was added, and the mixture was stirred at room temperature for 2 hours. To this solution, a saturated aqueous solution of sodium hydrogen carbonate was added, and extraction was performed three times using ethyl acetate. The obtained organic layer was washed with saturated saline and dried over anhydrous sodium sulfate. The solvent was distilled off from the organic layer. Thereafter, the residue was purified by silica gel column chromatography (solvent: hexane/ethyl acetate = 2/1), and thereby 460 mg of a compound C-1 as a white solid was obtained (yield: 60%).

NMR spectrum of compound C-1:

$^{1}$H-NMR (500 MHz, $CDCl_3$): δ7.36-7.29 (m, 6 H), 7.25-7.23 (m, 4 H), 5.36 (s, 2 H), 4.90 (br, 1 H), 3.43 (t, 2 H), 3.30 (m, 2 H), 1.80 (m, 2 H), 1.44 (s, 9 H) ppm.

To 3.7 g (9.2 mmol) of the compound C-1, 100 mL of THF and 1.4 g (9.2 mmol) of 4-chlorophenylisocyanate were added, and a gas phase was replaced with an argon gas. Thereafter, 2.8 g (28 mmol) of triethylamine was added, and the mixture was stirred at room temperature for 5 hours. The precipitated insoluble substance was separated by filtration, and the filtrate was concentrated. Dichloromethane was added to the concentrated filtrate, the precipitated insoluble substance was separated by filtration again, and the filtrate was concentrated. After this step was repeated three times, the solvent was distilled off from the filtrate. The obtained residue was purified by silica gel column chromatography (solvent: dichloromethane), and thereby 790 mg of a nitrile oxide compound C as a white solid was obtained (yield: 23%). Table 1 shows a melting point and an equivalent of nitrile oxide of the nitrile oxide compound C.

NMR spectrum of nitrile oxide compound C:

$^{1}$H-NMR (500 MHz, $CDCl_3$): δ7.42-7.33 (m, 10 H), 4.68 (br, 1 H), 3.52 (t, 2 H), 3.28 (m, 2 H), 1.87 (m, 2 H), 1.44 (s, 9 H) ppm.

Melting point of nitrile oxide compound C: 103°C

(Example 4A)

Synthesis of nitrile oxide compound D:

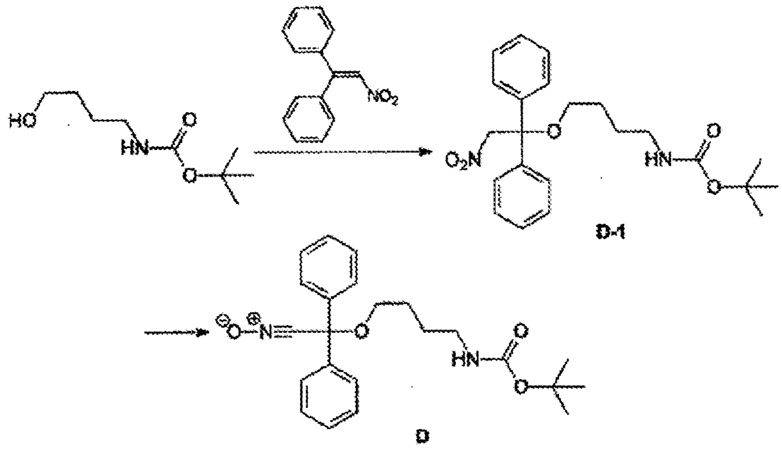

D-1

D

116 g (613 mmol) of 4-(tert-butoxycarbonylamino)-1-butanol was dissolved in 2000 mL of dehydrated THF, and cooled to 0°C. To this solution, 27.0 g (674 mmol) of sodium hydride was added under nitrogen gas, and the mixture was stirred at 0°C for 1 hour.

To this solution, 30 g (613 mmol) of 1-nitro-2,2-diphenylethylene was added, and the mixture was stirred at 20°C for 16 hours. The solution was cooled to 0°C, and thereafter was neutralized with an aqueous solution of 2 mol/L hydrogen chloride until a pH reached 6 to 7. The neutralized solution was extracted using dichloromethane. The dichloromethane solution was washed with saturated saline, dried over anhydrous sodium sulfate, and filtered. The filtrate was concentrated and was purified by column chromatography (acidic silica gel, solvent: petroleum ether/ethyl acetate 1/0, 1/1), and thereby 163 g of a compound D-1 as a yellow solid was obtained (yield: 64%).

NMR spectrum of compound D-1:

$^1$H-NMR (400 MHz, DMSO-$d_6$): δ7.36-7.28 (m, 8 H), 7.28-7.22 (m, 2 H), 6.80 (t, 1 H), 5.76 (s, 2 H), 3.33-3.29 (m, 2 H), 2.91 (q, 2 H), 1.57-1.43 (m, 4 H), 1.37 (s, 9 H) ppm.

60 g (144 mmol) of the compound D-1 was dissolved in 2000 mL of dehydrated dichloromethane. To this solution, 63 g (410 mmol) of 4-chlorophenylisocyanate, 82.9 g (819 mmol) of triethylamine, and 150 g of molecular sieves 4A were added, and the mixture was stirred at 20°C for 16 hours under nitrogen gas. The solution was filtered. The filtrate was concentrated and then purified by column chromatography (acidic silica gel, solvent: n-hexane/ethyl acetate 1/0, 4/1). Thereby, 10.6 g of a nitrile oxide compound D as a white solid was obtained (yield: 18%). Table 1 shows a melting point and an equivalent of nitrile oxide of the nitrile oxide compound D.

NMR spectrum of nitrile oxide compound D:

$^1$H-NMR (400 MHz, $CDCl_3$): δ7.47-7.29 (m, 10 H), 4.63 (brs, 1 H), 3.47 (t, 2 H), 3.14 (d, 2 H), 1.76-1.65 (m, 2 H), 1.65-1.55 (m, 2 H), 1.44 (s, 9 H) ppm.

Melting point of nitrile oxide compound D: 76°C

(Example 5A)

Synthesis of nitrile oxide compound E:

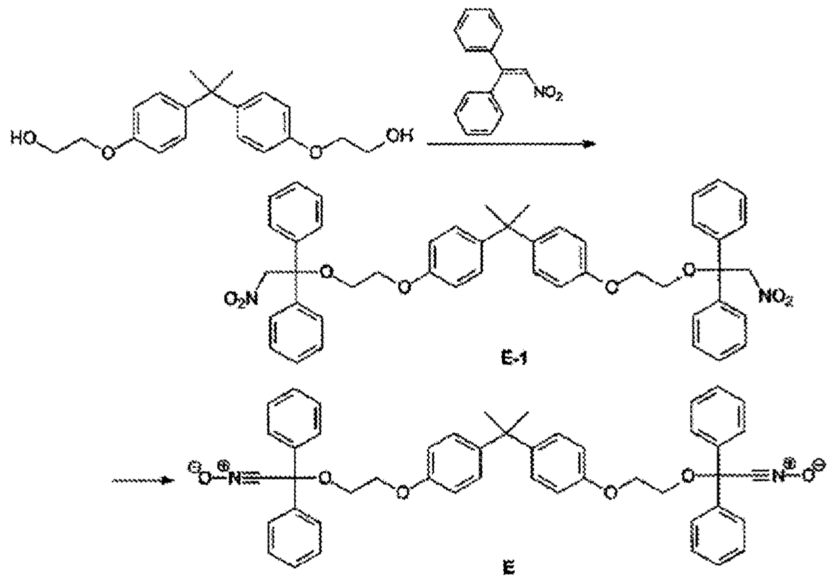

E-1

E

1.4 g (60 mmol) of sodium hydride was washed three times with hexane, a gas phase was replaced with an argon gas. Thereafter, 60 mL of DMF was added. To this solution, 3.2 g (10 mmol) of 4,4'-isopropylidenebis(2-phenoxyethanol) dissolved in 20 mL of DMF was slowly added at 0°C, and the mixture was stirred for 1 hour. To this solution, a DMF solution (20 mL) of 5.4 g (20 mmol) of 1-nitro-2,2-diphenylethylene was added dropwise at 0°C, and the mixture was stirred at room temperature for 4 hours. The reaction was stopped by adding a small amount of acetic acid to the solution, and extraction was performed using dichloromethane. The dichloromethane solution was washed with ion exchange water, a saturated aqueous solution of sodium hydrogen carbonate, and saturated saline, and was dried over anhydrous magnesium sulfate. The solvent was distilled off from the dichloromethane solution. Thereafter, the residue was purified by silica gel column chromatography (solvent: hexane/ethyl acetate = 2/1), and thereby 3.2 g of a compound E-1 as a yellow solid was obtained (yield: 42%).

NMR spectrum of compound E-1:

$^1$H-NMR (500 MHz, $CDCl_3$): δ734-7.26 (m, 20 H), 7.12 (d, 4 H), 6.79 (d, 4 H), 5.36 (s, 4 H), 4.17 (t, 4 H), 3.72 (t, 4 H), 1.63 (s, 6 H) ppm.

2.7 g (13 mmol) of di-tert-butyl dicarbonate and 50 mg (0.42 mmol) of N,N-dimethyl-4-aminopyridine were dissolved in 10 mL of acetonitrile under an argon gas atmosphere. To this solution, an acetonitrile solution (10 mL) of 3.2 g (4.2 mmol) of the compound E-1 was added, and the mixture was stirred at room temperature for 1 hour. The reaction was stopped by adding a small amount of acetic acid to the solution, and extraction was performed using ethyl acetate. The ethyl acetate solution was washed with a saturated aqueous solution of sodium hydrogen carbonate, a saturated aqueous solution of ammonium chloride, and saturated saline, and was dried over anhydrous magnesium sulfate. The solvent was distilled off from the ethyl acetate solution. Thereafter, the residue was purified by silica gel column chromatography (solvent: hexane/dichloromethane = 1/1), and thereby 1.2 g of a nitrile oxide compound E as a white solid was obtained (yield: 39%). Table 1 shows a melting point and an equivalent of nitrile oxide of the nitrile oxide compound E.

NMR spectrum of nitrile oxide compound E:

$^1$H-NMR (500 MHz, $CDCl_3$): δ7.45-7.33 (m, 20 H), 7.14 (d, 4 H), 6.83 (d, 4 H), 4.19 (t, 4 H), 3.83 (t, 4 H), 1.64 (s, 6 H) ppm.

Melting point of nitrile oxide compound E: 57°C

(Comparative Example 1A)

Synthesis of nitrile oxide compound F:

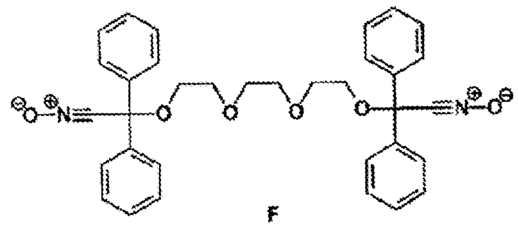

F

A nitrile oxide compound F as a pale yellow oil was obtained according to a method disclosed in Chemistry Letters, Vol. 46, 2017, p. 315-318. Table 1 shows an equivalent of nitrile oxide of the nitrile oxide compound F.

(Comparative Example 2A)

Synthesis of nitrile oxide compound G:

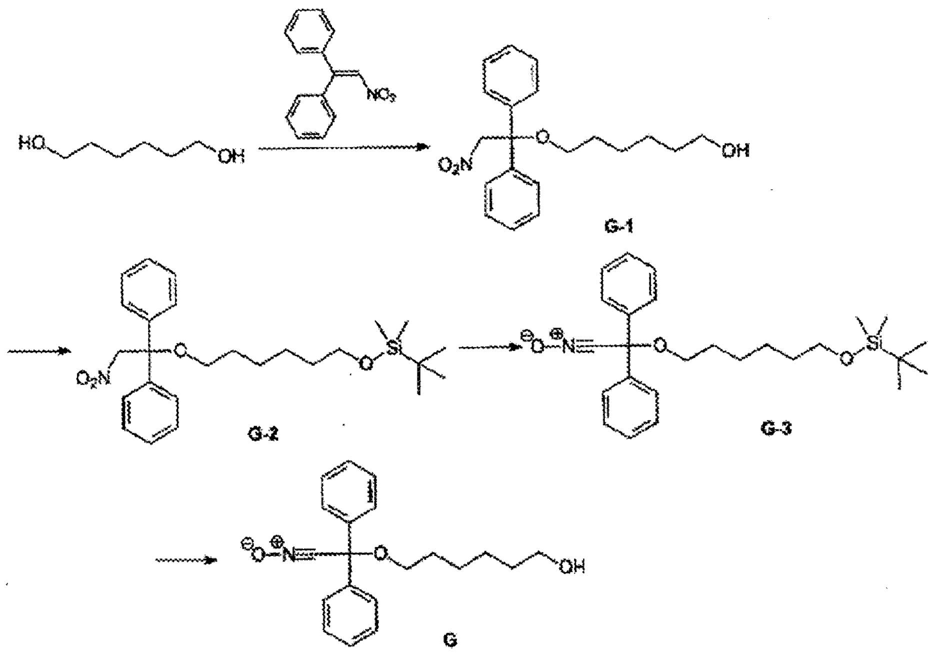

1.5 g (60 mmol) of sodium hydride was washed with hexane, and 40 mL of DMF was added. To this solution, 7.1 g (60 mmol) of 1,6-hexanediol dissolved in 10 mL of DMF was added dropwise at 0°C, and the mixture was stirred for 1 hour. To this solution, a DMF solution (10 mL) of 4.5 g (18 mmol) of 1-nitro-2,2-diphenylethylene was added dropwise, and the mixture was stirred for 16 hours. After a small amount of acetic acid was added to this solution, extraction was performed using dichloromethane. The dichloromethane solution was washed with water, a saturated aqueous solution of sodium hydrogen carbonate, and saturated saline, and was dried over anhydrous magnesium sulfate. The solvent was distilled off under reduced pressure from the dichloromethane solution. The residue was purified by silica gel column chromatography (solvent: hexane/ethyl acetate = 3/1), and thereby 4.7 g of a compound G-1 as a colorless viscous liquid was obtained (yield: 77%).

NMR spectrum of compound G-1:

$^1$H-NMR (500 MHz, $CDCl_3$): δ7.35-7.25 (m, 10 H), 5.34 (s, 2 H), 3.64 (dt, 2 H), 3.35 (t, 2 H), 1.66-1.36 (m, 8 H), 1.20 (t, 1 H) ppm.

4.7 g (14 mmol) of the compound G-1 and 1.1 g (16 mmol) of imidazole were dissolved in 30 mL of DMF. Thereafter, 2.3 g (15 mmol) of tert-butyldimethylchlorosilane was added dropwise, and the mixture was stirred at room temperature for 18 hours. The reaction was stopped by adding a saturated aqueous solution of ammonium chloride to the solution, and extraction was performed using ethyl acetate. The ethyl acetate solution was washed with water and

saturated saline, and was dried over anhydrous magnesium sulfate. The solvent was distilled off from the ethyl acetate solution. The residue was purified by silica gel column chromatography (solvent: hexane/ethyl acetate = 10/1), and thereby 5.6 g of a compound G-2 as a colorless viscous liquid was obtained (yield: 89%).

NMR spectrum of compound G-2:

$^1$H-NMR (500 MHz, $CDCl_3$): δ7.35-7.25 (m, 10 H), 5.34 (s, 2 H), 3.60 (t, 2 H), 3.35 (t, 2 H), 1.65-1.31 (m, 8 H), 0.89 (s, 9 H), 0.04 (s, 6 H) ppm.

5.5 g (12 mmol) of the compound G-2 was dissolved in 90 mL of dichloromethane. To this solution, 3.1 g (26 mmol) of phenyl isocyanate and 4.0 g (40 mmol) of triethylamine were added, and the mixture was stirred at room temperature for 2 hours. The solid was separated by filtration. Thereafter, the solvent was distilled off from the ethyl acetate solution. The residue was purified by silica gel column chromatography (solvent: hexane/ethyl acetate = 10/1), and thereby 5.2 g of a compound G-3 as a colorless viscous liquid was obtained (yield: 98%).

NMR spectrum of compound G-3:

$^1$ H-NMR (500 MHz, $CDCl_3$): δ7.42-7.31 (m, 10 H), 3.60 (t, 2 H), 3.45 (t, 2 H), 1.69-1.33 (m, 8 H), 0.89 (s, 9 H), 0.04 (s, 6 H) ppm.

5.2 g (12 mmol) of the compound G-3 was dissolved in 120 mL of THF. To this solution, 17 mL (17 mmol) of a THF solution of 1.0 mol/L tetra-n-butylammonium fluoride was added, and the mixture was stirred for 30 minutes. This solution was extracted using dichloromethane. The dichloromethane solution was washed with water and saturated saline, and was dried over anhydrous magnesium sulfate. The solvent was distilled off from the dichloromethane solution. The residue was purified by silica gel column chromatography (solvent: hexane/ethyl acetate = 3/1, 2/1), and thereby 1.4 g of a nitrile oxide compound G as a colorless viscous liquid was obtained (yield: 36%). Table 1 shows an equivalent of nitrile oxide of the nitrile oxide compound G.

NMR spectrum of nitrile oxide compound G:

$^1$ H-NMR (500 MHz, $CDCl_3$): δ7.42-7.32 (m, 10 H), 3.68-3.62 (m, 2 H), 3.47 (t, 2 H), 1.72-1.36 (m, 8 H), 1.24 (s, 1 H) ppm.

(Comparative Example 3A)

Synthesis of nitrile oxide compound H:

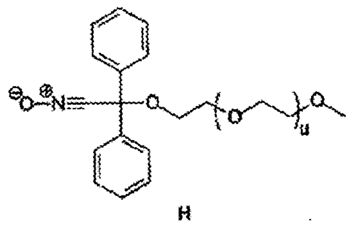

H

In Formula H, u is the number of repeating units.

A nitrile oxide compound H as a white solid was obtained according to a method disclosed in Polymer Chemistry, Vol. 8, 2017, p. 1445-1448 except that a molecular weight Mn of polyethylene glycol used as a raw material was changed from 3,100 to 2,000.

Table 1 shows a melting point and an equivalent of nitrile oxide of the nitrile oxide compound H.

(Comparative Example 4A)

Synthesis of nitrile oxide compound I:

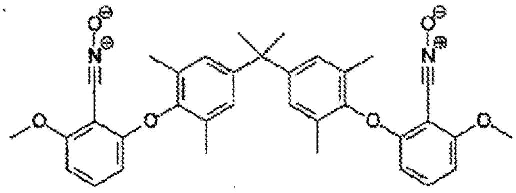

I

A nitrile oxide compound I as a yellow solid was obtained according to a method disclosed in Chemistry Letters, Vol. 39, 2010, p. 420-421. When a melting point of the nitrile oxide compound I was measured, the compound was decomposed at 250°C. Table 1 shows an equivalent of nitrile oxide of the nitrile oxide compound I.

<Evaluation of compounds>

(Storage stability)

The nitrile oxide compounds were put in a glass bottle. The bottle was sealed with a lid and allowed to stand at 30°C for 1 week. Thereafter, $^1$H-NMR was measured in $CDCl_3$. A case in which a spectrum was not changed before and after standing was indicated by "A," and a case in which a spectrum was changed was indicated by "B." Table 1 shows the results.

(Reactivity)

For evaluation of reactivity between a resin and the nitrile oxide compounds, "LABO PLASTOMILL μ," which was manufactured by Toyo Seiki Seisaku-sho, Ltd. and was equipped with a compact segment mixer KF6 (internal volume: 5 to 6 mL) and a type II blade having a 2 lobe disk shape (a combination of 5 disks), was used.

On an aluminum cup, 3.5 g (Mw: 58,300, Mn: 26,500, amount of end vinyl group: 34 μg/g) of powdered polypropylene having an end vinyl group was mixed with a predetermined amount of the nitrile oxide compound and 3.5 mg of an antioxidant (IRGANOX (registered trademark) 1010, manufactured by BASF), and thereby a sample was obtained. The solid nitrile oxide compound was mixed as it was. The oily nitrile oxide compound was dissolved in a small amount of acetone, sprinkled on polypropylene, and dried under reduced pressure at 30°C for 3 hours before use. An amount of the bifunctional nitrile oxide compound used was 2 equivalents of the end vinyl group, and an amount of the monofunctional nitrile oxide compound used was 1.2 equivalents of the end vinyl group.

The entire amount of the sample on the aluminum cup was put into the mixer of LABO PLASTOMILL preheated in advance to 180°C while rotating the blade at a low speed at 30 rpm, and the rotation speed of the blade was increased to 300 rpm to start the reaction. After 2 minutes, the rotation was stopped, and the sample was recovered.

In order to remove an unreacted nitrile oxide compound from the recovered sample, the sample was purified by the following method.

500 mg of the sample cut into several mm squares with scissors and 10 mL of dehydrated xylene were charged into a flask. Thereafter, the mixture was stirred at 120°C for 20 minutes under a nitrogen gas in an oil bath to be completely dissolved. 50 mL of acetone was put into this solution to precipitate a polymer. The precipitated polymer was recovered by filtration and dried at 60°C under reduced pressure for 4 hours or longer.

30 mg of the obtained polymer was dissolved in 0.7 mL of o-dichlorobenzene-$d_2$, and $^1$H-NMR was measured at 120°C. A reaction inversion rate was obtained from a ratio of a signal (2H) at 4.9 ppm which was derived from a vinyl group to a signal (1H) at about 4.5 ppm which was derived from an isoxazoline ring. Table 1 shows the results.

[Table 1]

| | Nitrile oxide compound | Meltingpoint (°C) | Equivalent of nitrile oxide (mmol/g) | Storage stability | Reaction inversion rate (%) |
|---|---|---|---|---|---|
| Example 1A | A | 135 | 3.96 | A | 100 |
| Example 2A | B | 95 | 3.76 | A | 100 |
| Example 3A | C | 103 | 2.61 | A | 83 |
| Example 4A | D | 76 | 2.52 | A | 85 |
| Example 5A | E | 57 | 2.74 | A | 100 |

(continued)

| | Nitrile oxide compound | Meltingpoint (°C) | Equivalent of nitrile oxide (mmol/g) | Storage stability | Reaction inversion rate (%) |
|---|---|---|---|---|---|
| Comparative Example 1A | F | Lower than 25°C | 3.54 | B | 100 |
| Comparative Example 2A | G | Lower than 25°C | 3.07 | B | 77 |
| Comparative Example 3A | H | 56 | 0.45 | A | 6 |
| Comparative Example 4A | I | Lower than 25°C | 3.46 | A | 0 |

It was found that the nitrile oxide compounds of Examples 1A to 5A respectively had a melting point of 25°C or higher and a large equivalent of nitrile oxide, and therefore the compounds had excellent storage stability and reactivity.

A melting point of each of the nitrile oxide compounds of Comparative Examples 1A to 2A was lower than 25°C, and therefore storage stability was poor.

The nitrile oxide compound of Comparative Example 3A had a melting point of 25°C or higher, but the compound had poor reactivity because of its high molecular weight and low equivalent of nitrile oxide.

The nitrile oxide compound of Comparative Example 4A had excellent storage stability but poor reactivity. The reason for this was because a reaction temperature was high, and a nitrile oxide group was isomerized into an isocyanate group.

(Example 6A)

Synthesis of nitrile oxide compound J:

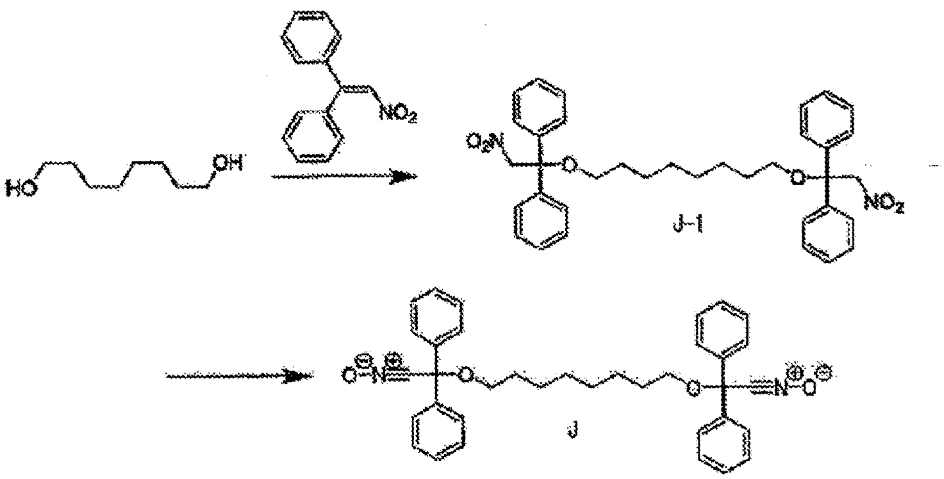


10.7 g (73.0 mmol) of 1,8-octanediol was dissolved in 200 mL of dehydrated THF, and cooled to 0°C. To this solution, 15 g (231 mmol) of sodium hydride (degree of purity: 37%) wad added under nitrogen gas, and the mixture was stirred at 0°C for 1 hour. To this solution, 35 g (155 mmol) of 1-nitro-2,2-diphenylethylene was added, and the mixture was stirred at 20°C for 15 hours. The solution was cooled to 0°C, and thereafter was neutralized with an aqueous solution of 2 mol/L hydrogen chloride until a pH reached 6 to 7. The neutralized solution was extracted using dichloromethane. The dichloromethane solution was washed with saturated saline, dried over anhydrous sodium sulfate, and filtered. The filtrate was concentrated and was purified by column chromatography (acidic silica gel, solvent: petroleum ether/ethyl acetate 1/0, 4/1), and thereby 31 g of a compound J-1 was obtained (yield: 68%).

NMR spectrum of compound J-1:

$^1$H-NMR (400 MHz, $CDCl_3$): δ7.38-7.27 (m, 18 H), 5.36 (s, 4 H), 3.36 (t, J=6.4 Hz, 4 H), 1.67-1.60 (m, 4 H), 1.42-1.34 (m, 4 H), 1.29 (brdd, J=3.6, 6.8 Hz, 4 H) ppm.

31 g (52.0 mmol) of the compound J-1 was dissolved in 500 mL of dehydrated dichloromethane. To this solution, 27.0 mL (211 mmol) of 4-chlorophenylisocyanate, 112.7 g (1.11 mol) of triethylamine, and 60 g of molecular sieves 4A were added, and the mixture was stirred at 20°C for 15 hours under nitrogen gas. The solution was filtered. The filtrate was concentrated and then purified by column chromatography (acidic silica gel, solvent: n-hexane/ethyl acetate 1/0, 10/1). Thereby, 8.1 g of a nitrile oxide compound J as a white solid was obtained (yield: 28%).

NMR spectrum of nitrile oxide compound B:

$^1$H-NMR (400 MHz, $CDCl_3$): δ=7.47-7.41 (m, 8 H), 7.40-7.31 (m, 12 H), 3.47 (t, J=6.4 Hz, 4 H), 1.75-1.63 (m, 4 H), 1.47-1.37 (m, 4 H), 1.36-1.29 (m, 4 H) ppm.

Melting point of nitrile oxide compound J: 86°C

(Example 7A)

Synthesis of nitrile oxide compound K:

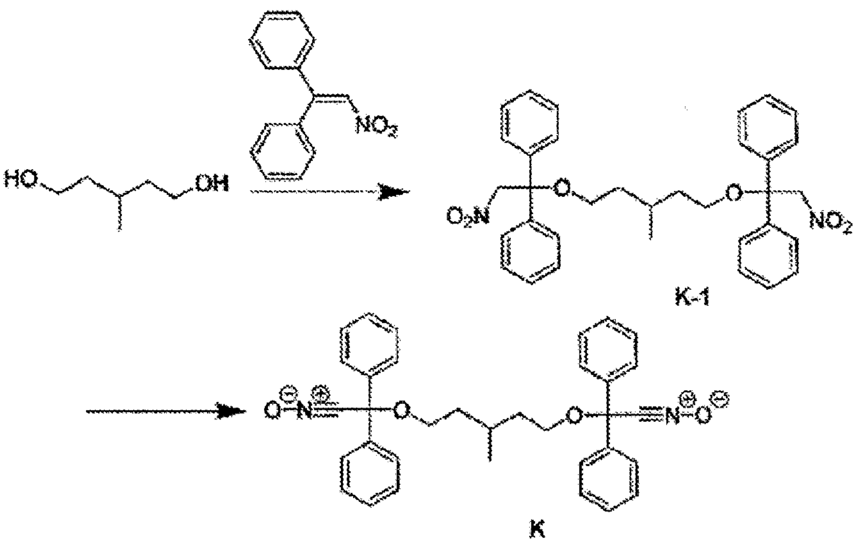

12.59 g (107 mmol) of 3-methyl-1,5-pentanediol was dissolved in 300 mL of dehydrated THF, and cooled to 0°C. To this solution, 13.32 g (333 mmol) of sodium hydride (degree of purity: 60%) wad added under nitrogen gas, and the mixture was stirred at 0°C for 1 hour. To this solution, 50 g (222 mmol) of 1-nitro-2,2-diphenylethylene was added, and the mixture was stirred at 15°C for 15 hours. The solution was cooled to 0°C, and thereafter was neutralized with an aqueous solution of 4 mol/L hydrogen chloride until a pH reached 4 to 5. The neutralized solution was extracted using dichloromethane. The dichloromethane solution was washed with saturated saline, dried over anhydrous sodium sulfate, and filtered. The filtrate was concentrated and was purified by column chromatography (acidic silica gel, solvent: petroleum ether/ethyl acetate 1/0, 5/1), and thereby 60 g of a compound K-1 was obtained (yield: 82%).

NMR spectrum of compound K-1:

$^1$H-NMR (400 MHz, $CDCl_3$) δ=7.36-7.22 (m, 20 H), 5.40-5.30 (m, 4 H), 3.48-3.33 (m, 4 H), 1.99-1.82 (m, 1 H), 1.69 (qd, J=6.4, 13.2 Hz, 2 H), 1.45 (qd, J=6.4, 13.5 Hz, 2 H), 0.84 (d, J=6.4 Hz, 3 H) ppm.

32 g (56 mmol) of the compound K-1 was dissolved in 900 mL of dehydrated dichloromethane. To this solution, 28.8 mL (225 mmol) of 4-chlorophenylisocyanate, 34.18 g (338 mmol) of triethylamine, and 25 g of molecular sieves 4A were added, and the mixture was stirred at 15°C for 16 hours under nitrogen gas. The solution was filtered. The filtrate was concentrated and then purified by column chromatography (acidic silica gel, solvent: n-hexane/ethyl acetate 1/0,

10/1). Thereby, 6.5 g of a nitrile oxide compound K as a white solid was obtained (yield: 22%).

NMR spectrum of nitrile oxide compound K:

$^1$H-NMR (400 MHz, $CDCl_3$): δ7.44-7.30 (m, 20 H), 3.45 (t, 4 H), 1.69 (m4 H), 1.41 (m, 4 H) ppm.
Melting point of nitrile oxide compound K: 103°C

(Example 8A)

Synthesis of nitrile oxide compound L:

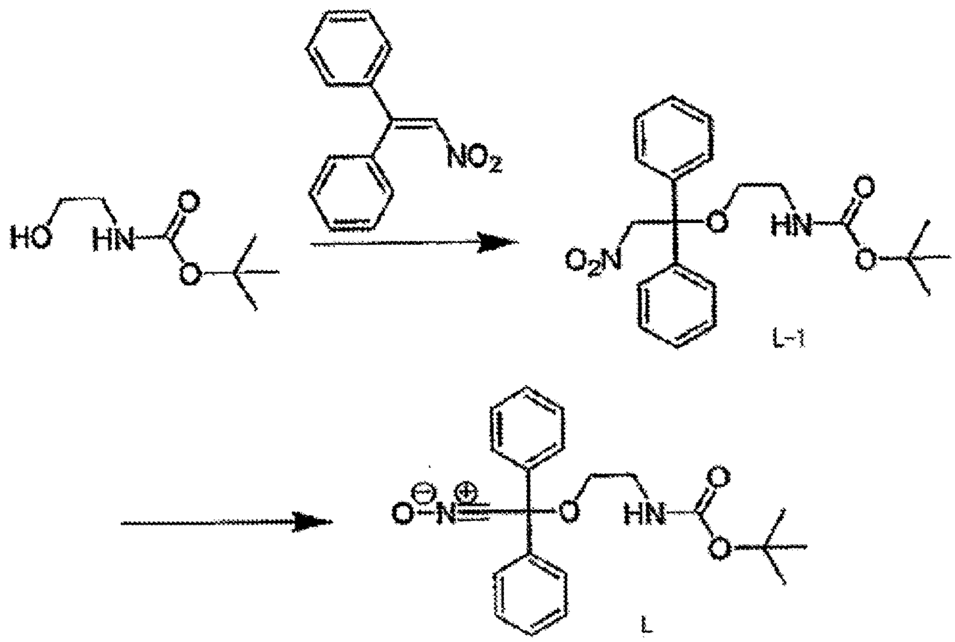

L-1

L

6.44 g (40.0 mmol) of 2-(tert-butoxycarbonylamino)-1-ethanol was dissolved in 60 mL of dehydrated THF, and cooled to 0°C. To this solution, 1.92 g (48.0 mmol) of sodium hydride (degree of purity: 60%) wad added under nitrogen gas, and the mixture was stirred at 0°C for 1 hour. To this solution, 9 g (40.0 mmol) of 1-nitro-2,2-diphenylethylene was added, and the mixture was stirred at 15°C for 15 hours. The solution was cooled to 0°C, and thereafter was neutralized with an aqueous solution of 1 mol/L hydrogen chloride until a pH reached 5 to 6. The neutralized solution was extracted using dichloromethane. The dichloromethane solution was washed with saturated saline, dried over anhydrous sodium sulfate, and filtered. The filtrate was concentrated. Thereafter, the obtained solid was washed with a mixed solution of petroleum ether/ethyl acetate (10/1), and thereby 13 g of a compound L-1 was obtained (yield: 84%).

NMR spectrum of compound L-1:

$^1$ H-NMR (400 MHz, $CDCl_3$) δ=7.37-7.30 (m, 6 H), 7.26-7.21 (m, 4 H), 5.33 (s, 2 H), 5.03 (brs, 1 H), 3.47-3.29 (m, 4 H), 1.45 (s, 9 H) ppm.
13 g (33.6 mmol) of the compound L-1 was dissolved in 250 mL of dehydrated dichloromethane. To this solution, 12.9 mL (101 mmol) of 4-chlorophenylisocyanate, 20.4 g (202 mmol) of triethylamine, and 25 g of molecular sieves 4A were added, and the mixture was stirred at 15°C for 16 hours under nitrogen gas. The solution was filtered. The filtrate was concentrated and then purified by column chromatography (acidic silica gel, solvent: n-hexane/ethyl acetate 1/0, 9/1). Thereby, 5.0 g of a nitrile oxide compound K as a white solid was obtained (yield: 40%).

NMR spectrum of nitrile oxide compound L:

$^1$ H-NMR (400 MHz, $CDCl_3$): δ7.46-7.34 (m, 10 H), 4.84 (brs, 1 H), 3.57-3.50 (m, 2 H), 3.47 (brd, J=5.2 Hz, 2 H), 1.45 (s, 9 H) ppm.

Melting point of nitrile oxide compound L: 76°C

(Example 9A)

Synthesis of nitrile oxide compound M:

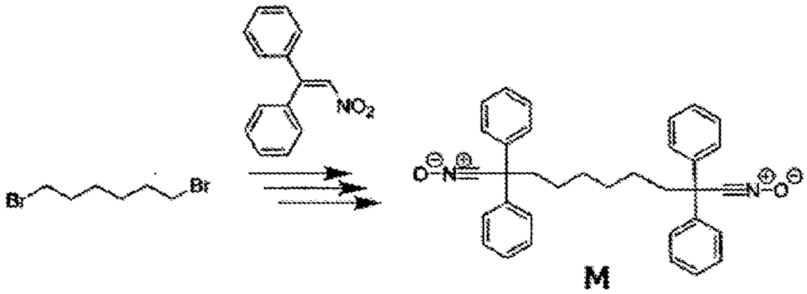

M

100 mL of dehydrated THF was put into 19.92 g (820 mmol) of metallic magnesium, a temperature was raised to 50°C under nitrogen, and thereafter, 650 mg (2.56 mmol) of iodine was added. After cooling to 15°C, a THF solution (100 ml) of 25 g (102.5 mmol) of 1,6-dibromohexane was added dropwise over 30 minutes, and the mixture was stirred at 15°C for 1 hour. A THF solution (100 m) of 28 g (124 mmol) of 1-nitro-2,2-diphenylethylene was added thereto, and the mixture was stirred at 15°C for 15 hours. The reaction solution was put into 285 g of sulfuric acid cooled to 0°C, and the mixture was stirred at 15°C for 30 minutes. This solution was separated with ethyl acetate/water. An organic phase was washed with an aqueous solution of sodium hydrogen carbonate and saturated saline, and dried over anhydrous sodium sulfate, and filtered. The filtrate was concentrated. The obtained solid was washed with a mixed solution of hexane/acetonitrile (1/1), and then purified by column chromatography (silica gel, solvent: dichloromethane/ethyl acetate (3/1)). Thereby, 10.9 g of a compound M was obtained (yield: 21%).

NMR spectrum of compound M-1:

$^1$ H-NMR (400 MHz, $CDCl_3$) $\delta$=7.40-7.22 (m, 20 H), 2.34 (brs, 4 H), 1.31 (s, 8 H) ppm.
Melting point of nitrile oxide compound M: 162°C

(Example 10A)

Synthesis of nitrile oxide compound N:

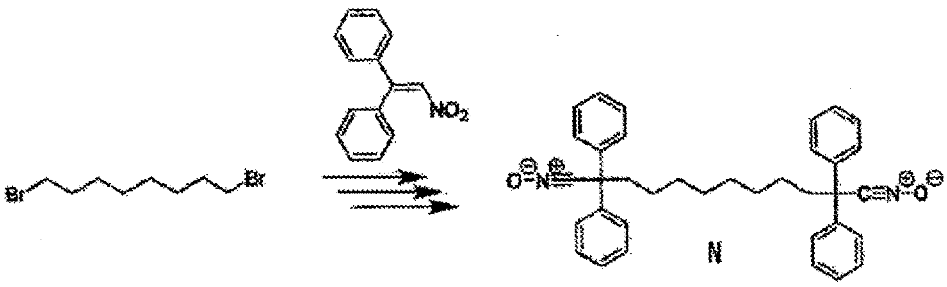

N

100 mL of dehydrated THF was put into 10.0 g (412 mmol) of metallic magnesium, a temperature was raised to 50°C under nitrogen, and thereafter, 327 mg (1.29 mmol) of iodine was added. After cooling to 15°C, a THF solution (100 ml) of 14 g (51.5 mmol) of 1,8-dibromohexane was added dropwise over 30 minutes, and the mixture was stirred at 15°C for 1 hour. A THF solution (100 m) of 14.0 g (62.2 mmol) of 1-nitro-2,2-diphenylethylene was added thereto, and the mixture was stirred at 20°C for 15 hours. The reaction solution was put into 368 g of sulfuric acid cooled to 0°C, and the mixture was stirred at 20°C for 1 hour. This solution was separated with dichloromethane-water. An organic

phase was washed with an aqueous solution of sodium hydrogen carbonate and saturated saline, and dried over anhydrous sodium sulfate, and filtered. The filtrate was concentrated. The obtained solid was washed with acetonitrile, and then purified by column chromatography (acidic silica gel, solvent: hexane/dichloromethane (1/1)). Thereby, 10.9 g of a compound N was obtained (yield: 14%).

NMR spectrum of compound N:

$^{1}$H-NMR (400 MHz, $CDCl_3$) $\delta$=7.40-7.22 (m, 20 H), 2.34 (brs, 4 H), 1.31 (s, 8 H) ppm.
Melting point of nitrile oxide compound N: 119°C
Each of the obtained nitrile oxide compounds J to N was evaluated. Table 2 shows the results.

[Table 2]

| | Nitrile oxide compound | Melting point (°C) | Equivalent of nitrile oxide (mmol/g) | Storage stability | Reaction inversion rate (%) |
|---|---|---|---|---|---|
| Example 6A | J | 86 | 3.57 | A | 100 |
| Example 7A | K | 103 | 3.75 | A | 100 |
| Example 8A | L | 76 | 2.71 | A | 84 |
| Example 9A | M | 162 | 4.00 | A | 84 |
| Example 10A | N | 119 | 3.78 | A | 85 |

It was found that, as in the case of the compounds of Examples 1A to 5A, the nitrile oxide compounds of Examples 6A to 10A also respectively had a melting point of 25°C or higher and a large equivalent of nitrile oxide, and therefore the compounds had excellent storage stability and reactivity.

<One-end-double-bonded polyolefin>

(PP1: propylene homopolymer)

Mw = 58,300, Mn = 26,500, Mw/Mn = 2.20

Tm = 156°C

Number of end vinyl groups = 34 μmol/g, 0.91 bonds/molecule

(PP2: propylene homopolymer)

Mw = 152,000, Mn = 59,600, Mw/Mn = 2.55

Tm = 154°C

Number of end vinyl groups = 17 μmol/g, 0.95 bonds/molecule

(PP3: propylene homopolymer)

Mw = 266,000, Mn = 76,100, Mw/Mn = 3.49
Tm = 153°C, MFR (230°C) = 9.2 g/10 min
Number of end vinyl groups = 10 μmol/g, 0.77 bonds/molecule

(PE1: ethylene-propylene copolymer)

Mw = 37,300, Mn = 13,800, Mw/Mn = 2.70
Tm = 66°C, MFR (190°C) = 107 g/10 min
Density = 0.889 g/cm$^3$
Ratio of propylene units: 14.8 mol%
Number of end vinyl groups = 51 μmol/g, 0.71 bonds/molecule
Number of vinylidene groups = 49 μmol/g, 0.13 bonds/molecule
Number of vinylene groups = 34 μmol/g, 0.47 bonds/molecule
Number of trisubstituted olefins = 33 μmol/g, 0.45 bonds/molecule

(PE2: ethylene-propylene-1-hexene copolymer)

Mw = 50,800, Mn = 19,100, Mw/Mn = 2.66
Tm = 54°C, MFR (190°C) = 30 g/10 min
Density = 0.874 g/cm$^3$
Ratio of propylene units: 9.4 mol%, ratio of hexene units: 4.9 mol%
Number of end vinyl groups = 17 μmol/g, 0.33 bonds/molecule
Number of vinylidene groups = 26 μmol/g, 0.49 bonds/molecule
Number of vinylene groups = 18 μmol/g, 0.34 bonds/molecule
Number of trisubstituted olefins = 24 μmol/g, 0.46 bonds/molecule

<Reactive-functional-group-containing polymer>

(EPDM: ethylene-propylene-dicyclopentadiene rubber)

Ratio of ethylene units: 57 wt%
Ratio of propylene units: 39 wt%
Ratio of dicyclopentadiene units: 4 wt%
Mooney viscosity ML (1 + 4) at 125°C: 48

<Production of modified polyolefins>

(Examples 1B, 3B to 5B, 7B, 10B, 13B to 20B, 23B, 24B, 28B, 30B, and 32B)

For production of modified polyolefins, "LABO PLASTOMILL μ," which was manufactured by Toyo Seiki Seisaku-sho, Ltd. and was equipped with a compact segment mixer KF6 (internal volume: 5 to 6 mL) and a type II blade having a 2 lobe disk shape (a combination of 5 disks), was used.

On an aluminum cup, 3.5 g of a one-end-double-bonded polyolefin was mixed with a predetermined amount of the nitrile oxide compound and 3.5 mg of an antioxidant (IRGANOX (registered trademark) 1010, manufactured by BASF), and thereby a mixture was obtained. The solid nitrile oxide compound was mixed as it was. The oily nitrile oxide compound was dissolved in a small amount of acetone, sprinkled on polyolefin, and dried under reduced pressure at 30°C for 3 hours before use. An amount of the nitrile oxide compound used was an equivalent shown in Table 3 with respect to a carbon-carbon double bond of the one-end-double-bonded polyolefin.

The entire amount of the mixture on the aluminum cup was put into the mixer of LABO PLASTOMILL preheated in advance to a kneading execution temperature while rotating the blade at a low speed at 30 rpm, and the rotation speed of the blade was increased to 300 rpm to start the reaction. After 2 minutes, the rotation was stopped, and the kneaded product was recovered.

In order to remove an unreacted nitrile oxide compound from the recovered kneaded product, the sample was purified by the following method.

500 mg of the kneaded product cut into several mm squares with scissors and 10 mL of dehydrated xylene were charged into a flask. Thereafter, the mixture was stirred at 120°C for 20 minutes under a nitrogen gas in an oil bath to be completely dissolved. 50 mL of acetone was put into this solution to precipitate a modified polyolefin.

The precipitated modified polyolefin was recovered by filtration and dried at 60°C under reduced pressure for 4 hours or longer. The results for reaction inversion rates are shown in Table 3. Fig. 1 is a $^1$H-NMR spectrum of a modified polyolefin of Example 14B.

(Examples 2B, 6B, 8B, 9B, 11B, 12B, 21B, 22B, 25B to 27B, 29B, and 31B)

Into a glass flask, 1.0 g of the one-end-double-bonded polyolefin, a predetermined amount of a nitrile oxide compound, and 10 mL of dehydrated xylene were put and heated and stirred at 110°C for 5 hours under nitrogen gas. An amount of the nitrile oxide compound used was an equivalent shown in Table 3 with respect to a carbon-carbon double bond of the one-end-double-bonded polyolefin. While cooling, 40 mL of acetone was added to this solution to precipitate a modified polyolefin. The precipitated modified polyolefin was recovered by filtration and dried at 60°C under reduced pressure for 4 hours or longer. The results for reaction inversion rates are shown in Table 3.

[Table 3]

| | One-end-double-bonded polyolefin | Nitrile oxide compound | | Reaction system | Reaction temperature (°C) | Vinyl inversion rate (%) | Vinylidene inversion rate (%) |
|---|---|---|---|---|---|---|---|
| | | Type | Equivalent | | | | |
| Example 1B | PP1 | A | 2.0 | Kneading | 180 | 100 | - |
| Example 2B | PP1 | A | 2.0 | Solution | 110 | 100 | - |
| Example 3B | PP3 | A | 2.0 | Kneading | 180 | 100 | - |
| Example 4B | PP3 | A | 1.5 | Kneading | 180 | 100 | - |
| Example 5B | PP3 | A | 1.2 | Kneading | 180 | 100 | - |
| Example 6B | PP3 | A | 2.0 | Solution | 110 | 100 | - |
| Example 7B | PP1 | B | 2.0 | Kneading | 180 | 100 | - |
| Example 8B | PP1 | B | 2.0 | Solution | 110 | 100 | - |
| Example 9B | PP3 | B | 2.0 | Solution | 110 | 100 | - |
| Example 10B | PP3 | B | 2.0 | Kneading | 180 | 100 | - |
| Example 11B | PE1 | C | 2.0 | Solution | 110 | 100 | 100 |
| Example 12B | PE2 | C | 2.0 | Solution | 110 | 100 | 85 |
| Example 13B | PE2 | C | 1.2 | Kneading | 180 | 100 | 74 |
| Example 14B | PP1 | C | 1.2 | Kneading | 180 | 83 | - |

(continued)

| | One-end-double-bonded polyolefin | Nitrile oxide compound | | Reaction system | Reaction temperature (°C) | Vinyl inversion rate (%) | Vinylidene inversion rate (%) |
|---|---|---|---|---|---|---|---|
| | | Type | Equivalent | | | | |
| Example 15B | PP3 | C | 1.2 | Kneading | 180 | 75 | - |
| Example 16B | PP3 | C | 1.2 | Kneading | 180 | 78 | - |
| Example 17B | PP3 | C | 1.6 | Kneading | 180 | 81 | - |
| Example 18B | PP3 | C | 1.2 | Kneading | 210 | 74 | - |
| Example 19B | PP3 | D | 2.0 | Kneading | 180 | 100 | - |
| Example 20B | PP3 | E | 2.0 | Kneading | 180 | 100 | - |
| Example 21B | PP1 | F | 2.0 | Solution | 110 | 100 | - |
| Example 22B | PP1 | F | 10.0 | Solution | 110 | 100 | - |
| Example 23B | PP1 | F | 2.0 | Kneading | 180 | 100 | - |
| Example 24B | PP2 | F | 2.0 | Kneading | 180 | 100 | - |
| Example 25B | PP2 | F | 2.0 | Solution | 110 | 100 | - |
| Example 26B | PP2 | F | 5.0 | Solution | 110 | 100 | - |
| Example 27B | PP3 | F | 2.0 | Solution | 110 | 100 | - |
| Example 28B | PP3 | F | 2.0 | Kneading | 180 | 100 | - |
| Example 29B | PP1 | G | 2.0 | Solution | 110 | 70 | - |
| Example 30B | PP1 | G | 1.2 | Kneading | 180 | 77 | - |
| Example 31 B | PP3 | G | 2.0 | Solution | 110 | 87 | - |
| Example 32B | PP3 | G | 1.2 | Kneading | 180 | 83 | - |

<Production of block copolymers>

(Examples 33B and 34B, and Comparative Examples 1B and 2B)

For production of block copolymers, "LABO PLASTOMILL μ," which was manufactured by Toyo Seiki Seisaku-sho, Ltd. and was equipped with a compact segment mixer KF6 (internal volume: 5 to 6 mL) and a type II blade having a 2 lobe disk shape (a combination of 5 disks), was used.

A modified polyolefin and a reactive-functional-group-containing polymer shown in Table 4, and 3.5 mg of an antioxidant (IRGANOX (registered trademark) 1010, manufactured by BASF) were put into the mixer of LABO PLASTOMILL preheated in advance to 180°C while rotating the blade at a low speed at 30 rpm, and the rotation speed of the blade was increased to 300 rpm to start the reaction. After 5 minutes, the rotation was stopped, and the kneaded product was recovered.

30 mg of the obtained kneaded product was dissolved in 0.7 mL of o-dichlorobenzene-d2, and $^1$H-NMR was measured at 120°C. In Examples 33B and 34B, signals at 5.05 ppm and 4.85 ppm of an isoxazoline ring which were generated by a nitrile oxide group were observed in addition to signals of a carbon-carbon double bond of a side chain of EPDM. Fig. 2 shows a $^1$H-NMR spectrum of a block copolymer of Example 33B.

A tensile test was performed on the obtained kneaded product. Table 4 shows the results.

[Table 4]

| | Modified polyolefin or one-end-double-bonded polyolefin | | Reactive-functional-group-containing polymer | | Tensile elastic modulus (MPa) | Yield stress (MPa) | Fracture stress (MPa) | Fracture strain (%) |
|---|---|---|---|---|---|---|---|---|
| Example 33B | Example 22B | 2.0 g | EPDM | 1.5 g | 380 | None | 14.3 | 440 |
| Comparative Example 1B | PP1 | 2.0 g | EPDM | 1.5 g | 244 | None | 2.85 | 2.8 |
| Example 34B | Example 26B | 2.0 g | EPDM | 1.5 g | 377 | 11.9 | 15.5 | 390 |
| Comparative Example 2B | PP2 | 2.0 g | EPDM | 1.5 g | 316 | 7.09 | 6.96 | 17 |

As shown in Table 3, it was found that by using the nitrile oxide compound, the functional group was introduced into the carbon-carbon double bond at the end of the polyolefin at a high reaction inversion rate.

By comparing the results of Example 33B and Comparative Example 1B, and the results of Example 34B and Comparative Example 2B in Table 4, it was found that the kneaded product of the modified polyolefin and EPDM was dramatically improved in a tensile elastic modulus, a fracture stress, and a fracture strain as compared to the kneaded product of the polyolefin in which the end had not been modified and EPDM. In particular, a fracture strain was significantly improved. In addition, when considering the results of NMR, it was found that the block copolymer was generated by the reaction of the nitrile oxide group at the end of the modified polyolefin with the carbon-carbon double bond of EPDM.

[Industrial Applicability]

By using the nitrile oxide compound of the present invention as a resin modifier or a rubber modifier, it is possible to obtain a modified resin or a modified rubber while excellent reactivity is exhibited at a high modification temperature that is industrially advantageous. Therefore, the nitrile oxide compound of the present invention is very useful from an industrial point of view.

According to the production method of the present invention, the end of the one-end-double-bonded polyolefin can be modified under industrially advantageous high-temperature conditions. In addition, the block copolymer having excellent mechanical properties can be produced by using the obtained modified polyolefin. Accordingly, the production method of the present invention has an extremely high industrial value.

## Claims

1. A nitrile oxide compound which is represented by General Formula [I], having a melting point of 25°C to 300°C, and having an equivalent of nitrile oxide of 1.0 to 4.5 mmol/g,

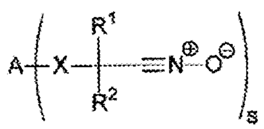

··· [I]

in General Formula [I],

s is an integer of 1 to 4,
$R^1$ and $R^2$ are each independently a hydrocarbon group having 4 to 10 carbon atoms or a halogenated hydrocarbon group having 4 to 10 carbon atoms,
X is a divalent hydrocarbon group, -O-, -S-, or -N($R^3$)-,
$R^3$ is a hydrogen atom or a hydrocarbon group having 1 to 6 carbon atoms, and
A is an s-valent organic group.

2. The nitrile oxide compound according to claim 1,
wherein, in General Formula [I], $R^1$ and $R^2$ are each independently an aryl group having 6 to 8 carbon atoms.

3. The nitrile oxide compound according to claim 1 or 2,
wherein, in General Formula [I],
s is 2, and
A is an alkylene group having 2 to 10 carbon atoms.

4. The nitrile oxide compound according to claim 3,
wherein, in General Formula [I],
A is a 1,2-ethylene group, a 1,3-propylene group, a 2-methyl-1,3-propylene group, a 2,2-dimethyl-1,3-propylene group, a 1,4-butylene group, a 1,5-pentylene group, a 1,6-hexylene group, a 1,7-heptylene group, a 1,8-octylene group, a 3-methyl-1,5-pentylene group, a 1,4-cyclohexylene group, a 1,4-cyclohexadimethylene group, a 1-methyl-1,2-ethylene group, or a 1-methyl-1,3-propylene group.

5. The nitrile oxide compound according to claim 1 or 2,
wherein, in General Formula [I],
s is 2, and
A is a group represented by General Formula [II]:

$$-(R^4\text{-}O)_m\text{-}R^5\text{-}(O\text{-}R^4)_m\text{-} \qquad [II]$$

in General Formula [II],

m is 0 or 1,
$R^4$ is an alkylene group having 2 to 4 carbon atoms, and
$R^5$ is a group represented by General Formula [III] or a group represented by General Formula [IV],

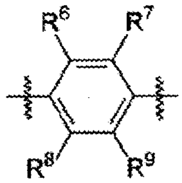

･･･[III]

in General Formula [III],
$R^6$ to $R^9$ are each independently a hydrogen atom, a hydrocarbon group having 1 to 6 carbon atoms, or a halogen atom, $R^6$ and $R^7$ may be linked to form an aromatic ring or an aliphatic ring, and $R^8$ and $R^9$ may be linked to form an aromatic ring or an aliphatic ring, and

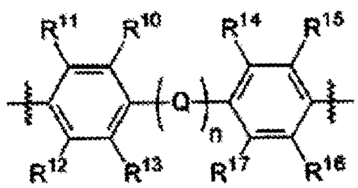

･･･[IV]

in General Formula [IV],

$R^{10}$ to $R^{17}$ are each independently a hydrogen atom, a hydrocarbon group having 1 to 6 carbon atoms, or a halogen atom, $R^{10}$ and $R^{11}$ may be linked to form an aromatic ring or an aliphatic ring, $R^{12}$ and $R^{13}$ may be linked to form an aromatic ring or an aliphatic ring, $R^{14}$ and $R^{15}$ may be linked to form an aromatic ring or an aliphatic ring, and $R^{16}$ and $R^{17}$ may be linked to form an aromatic ring or an aliphatic ring,
n is 0 or 1, and
Q is $-C(R^{18})(R^{19})-$, $-C(=O)-$, $-S-$, or $-S(=O)_2-$,
where $R^{18}$ and $R^{19}$ are each independently a hydrogen atom, a hydrocarbon group having 1 to 6 carbon atoms, or a halogen atom, and $R^{18}$ and $R^{19}$ may be linked to form an aromatic ring or an aliphatic ring.

**6.** The nitrile oxide compound according to claim 5,
wherein, in General Formula [II],
m is 1, and
$R^5$ is a group represented by General Formula [IV], and in General Formula [IV],
n is 1, and
Q is $-C(R^{18})(R^{19})-$.

**7.** The nitrile oxide compound according to claim 1 or 2,
wherein, in General Formula [I],
s is 1, and
A is a group represented by General Formula [V]:

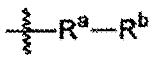

···[V]

in General Formula [V],

$R^a$ is an alkylene group having 1 to 5 carbon atoms or an arylene group having 6 to 10 carbon atoms, and
$R^b$ is a polar functional group.

**8.** The nitrile oxide compound according to claim 7,
wherein, in General Formula [V],
$R^b$ is a hydroxy group, a mercapto group, a carboxy group, an amino group, an amino group having a substituent, an amide group, $-OR^{20}$ (where $R^{20}$ is an alkyl group or an aryl group), or a heterocyclic ring.

**9.** A composition comprising:

the nitrile oxide compound according to any one of claims 1 to 8; and
a substance that can react with a nitrile oxide group of the nitrile oxide compound.

**10.** The composition according to claim 9,
wherein the substance that can react with a nitrile oxide group is a resin or a rubber.

**11.** The composition according to claim 10,
wherein the resin or the rubber has at least one bond selected from the group consisting of a carbon-carbon double bond, a carbon-carbon triple bond, a carbon-nitrogen double bond, and a carbon-nitrogen triple bond.

**12.** A method for producing a modified polyolefin, the method comprising:
performing an addition reaction of a polyolefin having a carbon-carbon double bond at one end of two ends of a main chain with a compound having a 1,3-dipolar functional group.

**13.** The method for producing a modified polyolefin according to claim 12,
wherein the compound having a 1,3-dipolar functional group is a nitrile oxide compound.

**14.** The method for producing a modified polyolefin according to claim 13,
wherein the nitrile oxide compound is a compound represented by General Formula [I]:

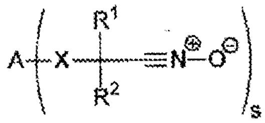

・・・[I]

in General Formula [I],

s is an integer of 1 to 4,
$R^1$ and $R^2$ are each independently a hydrocarbon group having 4 to 10 carbon atoms or a halogenated hydrocarbon group having 4 to 10 carbon atoms,
X is a divalent hydrocarbon group, -O-, -S-, or -N($R^3$)-,
$R^3$ is a hydrogen atom or a hydrocarbon group having 1 to 6 carbon atoms, and
A is an s-valent organic group.

**15.** The method for producing a modified polyolefin according to any one of claims 12 to 14, wherein the number of carbon-carbon double bonds per one polyolefin molecule in the polyolefin is 0.1 to 4.0 bonds/molecule.

**16.** The method for producing a modified polyolefin according to any one of claims 12 to 15, wherein the carbon-carbon double bond at the end of the main chain of the polyolefin is a vinyl group or a vinylidene group.

**17.** The method for producing a modified polyolefin according to any one of claims 12 to 16, further comprising: performing an addition reaction of the polyolefin with the compound having a 1,3-dipolar functional group under conditions substantially free of a solvent.

**18.** A modified polyolefin obtained by the production method according to any one of claims 12 to 17.

**19.** A modified polyolefin having a structure represented by General Formula [VIII]:

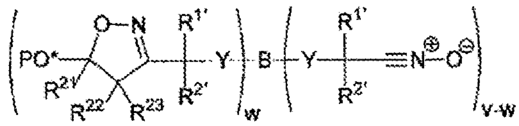

・・・[VIII]

in General Formula [VIII],

v is an integer of 1 to 4,
w is an integer of 1 to 4,
$v \geq w$,
PO* is a main chain of the polyolefin,
$R^{21}$, $R^{22}$, and $R^{23}$ are each independently a hydrogen atom or an alkyl group having 1 to 8 carbon atoms,
$R^{1'}$ and $R^{2'}$ are each independently a hydrocarbon group having 4 to 10 carbon atoms or a halogenated hydrocarbon group having 4 to 10 carbon atoms,
Y's are each independently a divalent hydrocarbon group, -O-, -S-, or -N($R^3$)-,
$R^{3'}$ is a hydrogen atom or a hydrocarbon group having 1 to 6 carbon atoms, and
B is a v-valent organic group.

**20.** The modified polyolefin according to claim 19, wherein, in General Formula [VIII], $R^{22}$ and $R^{23}$ are hydrogen atoms.

**21.** A method for producing a block copolymer, the method comprising: reacting the modified polyolefin according to any one of claims 18 to 20 with a polymer having a functional group that can react with a functional group of the modified polyolefin.

**22.** The method for producing a block copolymer according to claim 21, wherein the functional group that can react with a functional group of the modified polyolefin is at least one selected from the group consisting of a carbon-carbon double bond, a carbon-carbon triple bond, a carbon-nitrogen triple

bond, an acid anhydride group, and an amino group.

**23.** The method for producing a block copolymer according to claim 21 or 22, the method comprising: reacting the modified polyolefin with the polymer under conditions substantially free of a solvent.

**24.** The method for producing a block copolymer according to any one of claims 21 to 23, wherein a reaction temperature is 150°C or higher.

FIG. 1

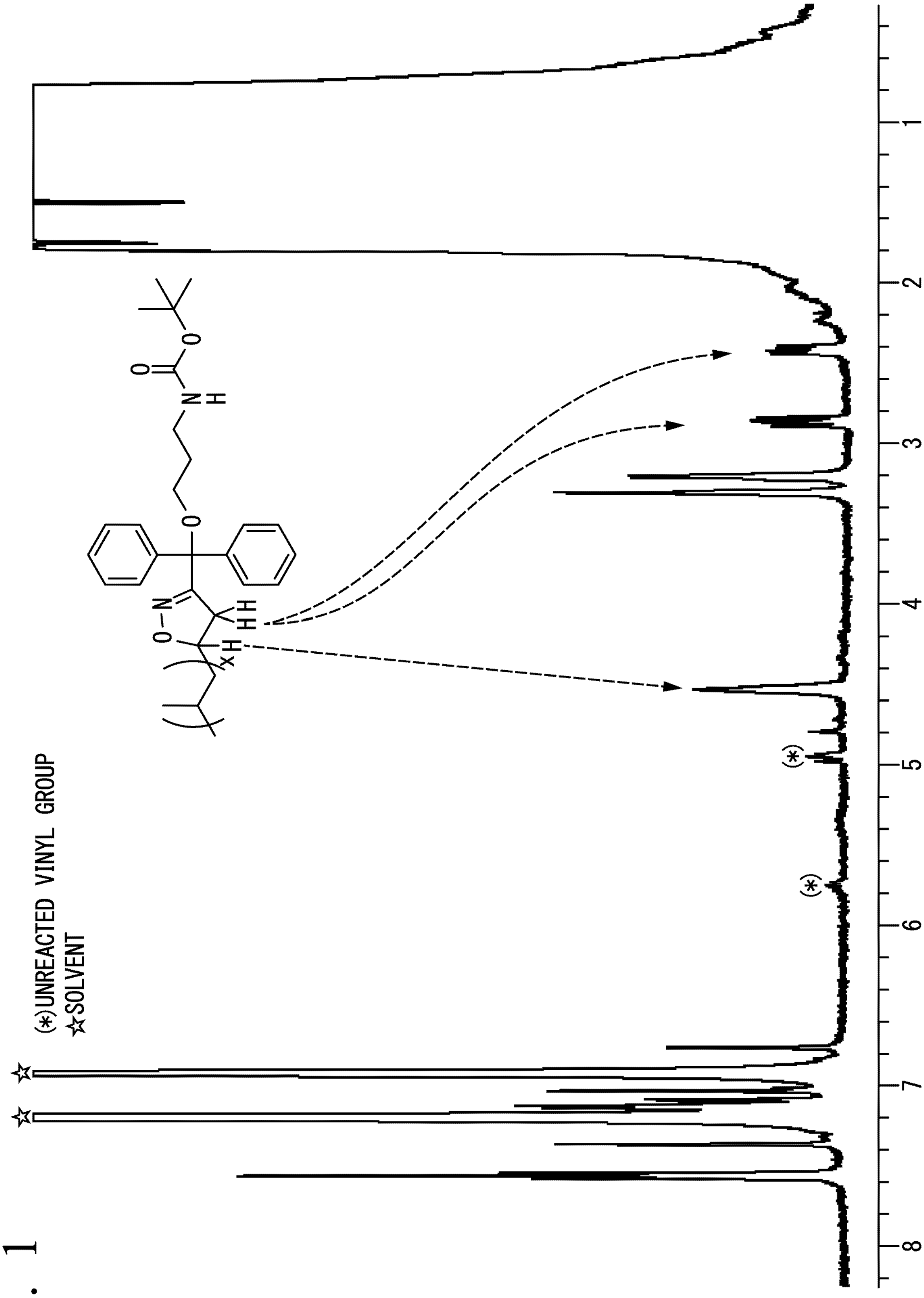

FIG. 2

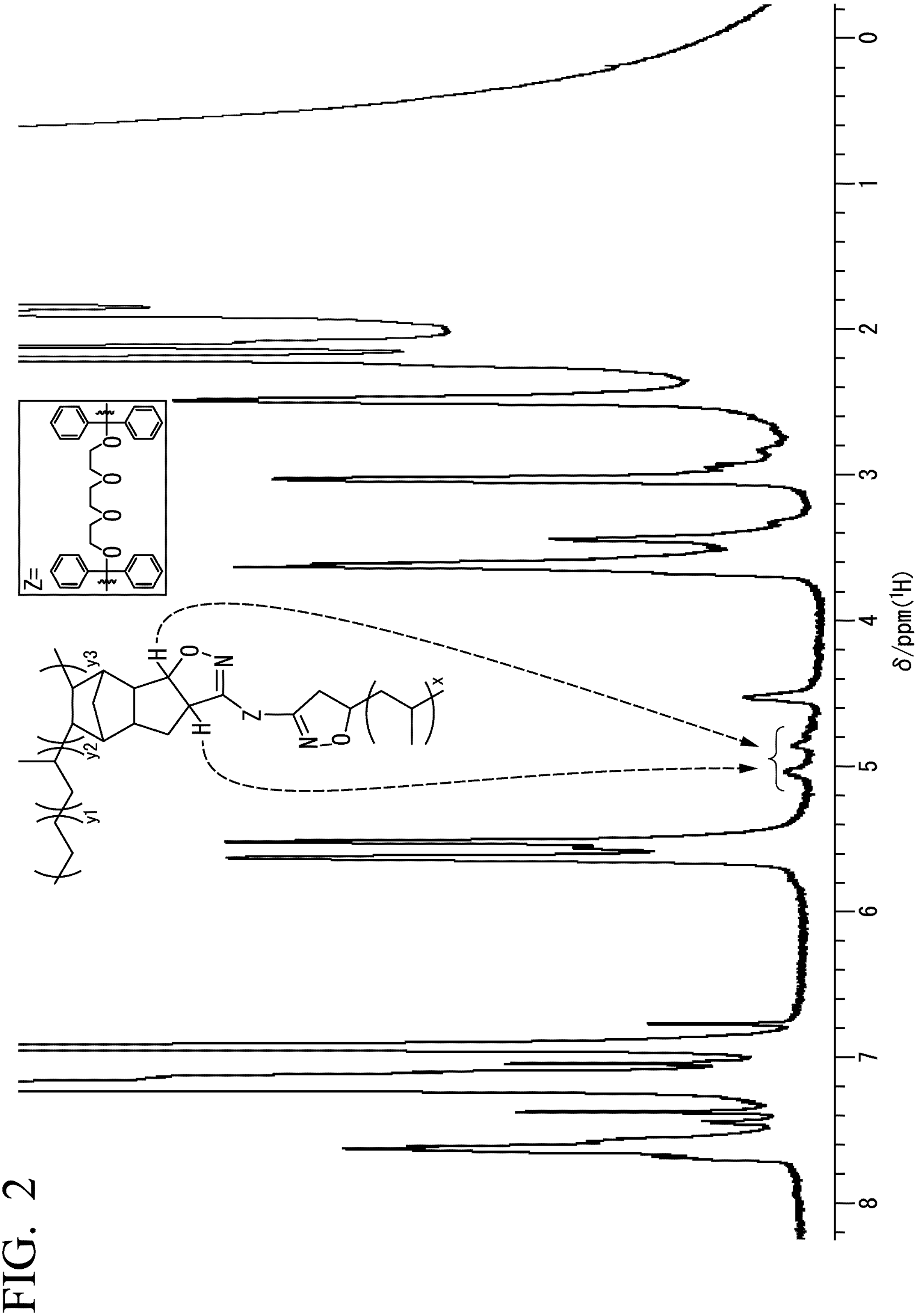

**INTERNATIONAL SEARCH REPORT**

International application No.
PCT/JP2018/043868

A. CLASSIFICATION OF SUBJECT MATTER
Int.Cl. C07C291/06(2006.01)i, C08FS/30(2006.01)i

According to International Patent Classification (IPC) or to both national classification and IPC

B. FIELDS SEARCHED
Minimum documentation searched (classification system followed by classification symbols)
Int.Cl. C07B, C07C, C08C, C08F

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| Y<br>A | JP 2013-112741 A (TOYOTA GOSEI CO., LTD.) 10 June 2013, claims, paragraphs [0047], [0075] (Family: none) | 12-18<br>1-11, 19-24 |
| Y<br>A | WO 2005/073282 A1 (MITSUI CHEMICALS, INC.) 11 August 2005, paragraph [0002] & EP 1719794 A1 paragraph [0002] | 12-18<br>1-11, 19-24 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 February 2019 (14.02.2019) | 26 February 2019 (26.02.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No.
PCT/JP2018/043868

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | JP 2001-2731 A (MITSUI CHEMICALS, INC.) 09 January 2001, claims, paragraph [0028] (Family: none) | 12-18<br>1-11, 19-24 |
| Y<br>A | JP 2003-73412 A (MITSUI CHEMICALS, INC.) 12 March 2003, claims, paragraphs [0004], [0005] & US 2003/0027955 A1 paragraphs [0004]-[0007] | 12-18<br>1-11, 19-24 |
| A | JP 2011-52072 A (TOYOTA GOSEI CO., LTD.) 17 March 2011, claims & US 2011/0054134 A1, claims | 1-24 |
| A | WO 2014/136952 A1 (DAIKIN INDUSTRIES, LTD.) 12 September 2014, claims & US 2016/0002153 A1, claims | 1-24 |
| A | YAO, Chingfa et al., "Generation of Nitroalkanes, Hydroximoyl Halides and Nitrile Oxides from the Reactions of β-Nitrostyrenes with Grignard or Organolithium Reagents", Tetrahedron, 1998, 54, pp. 791-822 | 1-24 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2017227833 A
- JP 2017227834 A
- JP 2016065033 A
- WO 2016143869 A
- WO 2016143870 A
- JP HEI11180943 B
- JP 2011052072 A


### Non-patent literature cited in the description


- **KOYAMA et al.** Nitrile Oxide Agents for Click Reaction: Catalyst-Free Cycloaddition Reaction Involving C-C Bond Formation. *Journal of Synthetic Organic Chemistry,* 2016, vol. 47 (9), 866-876
- **CHING-FA YAO et al.** REACTIONS OF β-NITROTYRENES WITH GRIGNARD REAGENTS. *Tetrahedron Letters,* 1996, vol. 37 (35), 6339-6342
- **TOYOKAZU TSUTSUBA et al.** Kinetically Stabilized Aliphatic Nitrile N-Oxides as Click Agents: Synthesis, Structure, and Reactivity. *Chemistry Letters,* 2017, vol. 46, 315-318
- **PATRICK BRANT et al.** *Termination Events in Sterically Hindered Metallocene-Catalyzed Olefin Oligomerizations: Vinyl Chain Ends in Oligooctenes,* 2016, vol. 35, 2836-2839
- *Macromolecules,* 2005, vol. 38, 6988-6996
- *Topics in Catalysis,* 1999, vol. 7, 145-163
- Thermoplastic Elastomers. Hanser Gardner Publications, 1996, 153-190
- *Journal of Polymer Science Part A: Polymer Chemistry,* 2004, vol. 42, 2474-2482
- *Chemical Communications,* 2013, vol. 49, 7723-7725
- *Chemistry Letters,* 2017, vol. 46, 315-318
- *Polymer Chemistry,* 2017, vol. 8, 1445-1448
- *Chemistry Letters,* 2010, vol. 39, 420-421